# EUROPEAN PATENT APPLICATION

(11) **EP 4 803 503 A1**
(43) Date of publication of application: **09.09.2026**
(21) Application number: 24885888.8
(22) Date of filing: 01.11.2024
(51) Int. Cl.: C07C 68/08, C07C 69/96

(54) **ETHYL METHYL CARBONATE PRODUCTION METHOD**

(30) Priority: 02.11.2023 JP 2023188509
(71) Applicant: ASAHI KASEI KABUSHIKI KAISHA, Tokyo 100-0006 (JP)
(72) Inventor: ENOMOTO, Miyako, Tokyo 100-0006 (JP)
(74) Representative: Strehl & Partner mbB
(86) International application number: PCT/JP2024/039151
(87) International publication number: WO 2025/095124

(57) **Abstract**

A method for producing ethyl methyl carbonate including: distillation step A of feeding a feed containing dimethyl carbonate, ethyl methyl carbonate, and ethanol to a distillation column A, and extracting a fraction A_{B} from a column bottom of the distillation column A, wherein the feed contains dimethyl carbonate in an amount of 25 mol% or more based on a whole amount of the feed and ethyl methyl carbonate in an amount of 12 mol% or more based on a whole amount of the feed, the feed has a molar ratio dimethyl carbonate/(ethanol + ethyl methyl carbonate) of 0.5 to 5.6, a proportion of ethanol to ethyl methyl carbonate in the fraction A_{B} is 20 ppm by mass or less, and a proportion of methanol to ethyl methyl carbonate in the fraction A_{B} is 20 ppm by mass or less.

## Description

### Technical Field

The present invention relates to a method for producing ethyl methyl carbonate.

### Background Art

Ethyl methyl carbonate that is used as an organic solvent for battery electrolytes is typically, widely known to be produced through a transesterification reaction of dimethyl carbonate and ethanol.

Ethyl methyl carbonate having a methanol concentration of 20 ppm by mass or less and an ethanol concentration of 20 ppm by mass or less as a standard is typically used as the battery electrolyte; however, since ethanol as a raw material and ethyl methyl carbonate as the final product have an azeotropic composition at ordinary pressure, rectification to achieve the aforementioned alcohol concentration was inefficient due to low energy efficiency and a large loss of ethyl methyl carbonate.

For example, Patent Literature 1 proposes a method for separating ethanol from ethyl methyl carbonate by using a pressurization column. Patent Literature 2 proposes a means for improving the energy efficiency required for purification of ethyl methyl carbonate by using a heat pump.

### Citation List

### Patent Literature

Patent Literature 1: CN212687934U
Patent Literature 2: CN110105216A

### Summary of Invention

### Technical Problem

In the purification of ethyl methyl carbonate by distillation, dimethyl carbonate and methanol; dimethyl carbonate and ethanol; and ethyl methyl carbonate and ethanol are known to form azeotropic compositions at ordinary pressure. In particular, the formation of an azeotropic composition of ethyl methyl carbonate and ethanol is problematic in obtaining high purity of ethyl methyl carbonate, and the purification has been carried out by methods with low energy efficiency to remove these alcohols, that is, methanol and ethanol.

In Patent Literature 1 and Patent Literature 2, methods for removing alcohols by eliminating the azeotrope of methyl ethyl carbonate and alcohols by pressurization are used; however, the temperature increases along with an increase in the boiling points of all the components due to pressurization, and the methods require a lot of heat sources, resulting in low energy efficiency.

Thus, an object of the present invention is to produce ethyl methyl carbonate having low ethanol and methanol contents with high efficiency.

### Solution to Problem

The present inventor has found that the aforementioned problem can be solved by allowing a predetermined amount or more of dimethyl carbonate to be present upon purification of ethyl methyl carbonate by distillation.

Specifically, the present invention encompasses the following embodiments.
<1> A method for producing ethyl methyl carbonate comprising:
   distillation step A of feeding a feed containing dimethyl carbonate, ethyl methyl carbonate, and ethanol to a distillation column A, and extracting a fraction A_{B} from a column bottom of the distillation column A, wherein
   the feed contains dimethyl carbonate in an amount of 25 mol% or more based on a whole amount of the feed and ethyl methyl carbonate in an amount of 12 mol% or more based on a whole amount of the feed,
   the feed has a molar ratio dimethyl carbonate/(ethanol + ethyl methyl carbonate) of 0.1 to 2.0, and
   a proportion of ethanol to ethyl methyl carbonate in the fraction A_{B} is 20 ppm by mass or less, and a proportion of methanol to ethyl methyl carbonate in the fraction A_{B} is 20 ppm by mass or less.
<2> The method for producing ethyl methyl carbonate according to <1>, further comprising:
   (1) distillation step B of feeding the fraction A_{B} to a distillation column B, and extracting a fraction B_{B} from which a low-boiling point substance having a lower boiling point than a boiling point of ethyl methyl carbonate has been removed, from a column bottom of the distillation column B, and
      distillation step C of feeding the fraction B_{B} to a distillation column C, and extracting a fraction C_{L} from a column upper part of the distillation column C,
      wherein ethyl methyl carbonate purity in the fraction C_{L} is 99.99% by mass or more,
      or
   (2) distillation step D of feeding the fraction A_{B} to a distillation column D, and extracting a fraction D_{B} from a column bottom of the distillation column D, a fraction D_{L} from a column top, and a fraction D_{S} from an intermediate part of the distillation column D,
      wherein ethyl methyl carbonate purity in the fraction D_{S} is 99.99% by mass or more,
      or
   (3) distillation step B of feeding the fraction A_{B} to a distillation column B, and extracting a fraction B_{B} from which a low-boiling point substance having a lower boiling point than a boiling point of ethyl methyl carbonate has been removed, from a column bottom of the distillation column B, and
      distillation step D of feeding the fraction B_{B} to a distillation column D, and extracting a fraction D_{B} from a column bottom of the distillation column D, a fraction D_{L} from a column top, and a fraction D_{S} from an intermediate part of the distillation column D,
      wherein ethyl methyl carbonate purity in the fraction D_{S} is 99.99% by mass or more.
<3> The method for producing ethyl methyl carbonate according to <1> or <2>, wherein a column top pressure in the distillation column A is 0 kPaG to 50 kPaG.
<4> The method for producing ethyl methyl carbonate according to any one of <1> to <3>, wherein the method for feeding at least a part of dimethyl carbonate in the feed to the distillation column A is a single addition of a liquid with a pump.
<5> The method for producing ethyl methyl carbonate according to any one of <1> to <4>, wherein the feed is introduced from a column intermediate part of the distillation column A.
<6> The method for producing ethyl methyl carbonate according to any one of <1> to <5>, wherein the feed contains ethanol in an amount of 0.5 mol% or more based on a whole amount of the feed.
<7> The method for producing ethyl methyl carbonate according to any one of <1> to <6>, wherein the distillation column A includes a tray and/or a filling as an internal.
<8> The method for producing ethyl methyl carbonate according to any one of <1> to <7>, further comprising, before the distillation step A:
   a reaction step of subjecting dimethyl carbonate and ethanol to a transesterification reaction in a reaction apparatus to obtain a reaction composition,
   wherein the reaction composition is to be fed to the distillation column A as at least a part of the feed.
<9> The method for producing ethyl methyl carbonate according to <2>, further comprising a step of distilling a fraction C_{B} or a fraction D_{B} extracted from a column bottom of the distillation column C or the distillation column D to obtain diethyl carbonate having a purity of 99.99% by mass or more.
<10> The method for producing ethyl methyl carbonate according to any one of <1> to <9>, further comprising:
   before the distillation step A, a reaction step of subjecting dimethyl carbonate and ethanol to a transesterification reaction in a reaction apparatus to obtain a reaction composition,
   distillation step B of feeding the fraction A_{B} to a distillation column B, and extracting a fraction B_{B} from which a low-boiling point substance having a lower boiling point than a boiling point of ethyl methyl carbonate has been removed, from a column bottom of the distillation column B, and
   distillation step C of feeding the fraction B_{B} to a distillation column C, and extracting a fraction C_{L} from a column upper part of the distillation column C,
   wherein the reaction composition is fed to the distillation column A as at least a part of the feed, and
   ethyl methyl carbonate purity in the fraction C_{L} is 99.99% by mass or more.

### Advantageous Effect of Invention

According to the present invention, ethyl methyl carbonate having low ethanol and methanol contents can be produced with high efficiency.

### Brief Description of Drawings

[Figure 1] Figure 1 is a schematic diagram of the production equipment used in the method for producing ethyl methyl carbonate according to the present embodiment.
[Figure 2] Figure 2 is a schematic diagram of the production equipment used in the method for producing ethyl methyl carbonate according to the present embodiment.
[Figure 3] Figure 3 is a schematic diagram of the production equipment used in the method for producing ethyl methyl carbonate according to the present embodiment.
[Figure 4] Figure 4 is a schematic diagram of the production equipment used in the method for producing ethyl methyl carbonate according to the present embodiment.
[Figure 5] Figure 5 is a schematic diagram of the production equipment used in the method for producing ethyl methyl carbonate according to the present embodiment.

### Description of Embodiments

Hereinafter, the embodiment of the present invention (hereinafter, referred to as "the present embodiment") will be described in detail with reference to the drawings as needed; however, the present invention is not limited to these, and can be variously modified without departing from the gist thereof. In the drawings, positional relationships such as up, down, left, and right are based on the positional relationships illustrated in the drawings, unless otherwise specified. The dimensional ratios in the drawings are not limited to the illustrated ratios.

Hereinafter, the meanings of the terms and abbreviations used in the present specification will be described.

The term "EMC" means ethyl methyl carbonate.

The term "DMC" means dimethyl carbonate.

The term "DEC" means diethyl carbonate.

The term "MeOH" means methanol.

The term "EtOH" means ethanol.

"A", "B", "C" and the like in the distillation column A, the distillation column B, the distillation column C, step A, step B, and step C are merely notations for describing distillation columns, and are not intended to limit the configuration, order, number, and others of the distillation columns.

A numerical range indicated by using "to" refers to a range including the numerical values described before and after "to" as the minimum value and the maximum value, respectively. In numerical ranges described in stages in the present specification, an upper limit value or a lower limit value in a numerical range of a certain stage may be arbitrarily combined with an upper limit value or a lower limit value in a numerical range of another stage.

The method for producing ethyl methyl carbonate according to the present embodiment has a distillation step of feeding a feed containing dimethyl carbonate, ethyl methyl carbonate, and ethanol to a distillation column, and extracting from the column bottom of the distillation column, wherein
the feed contains dimethyl carbonate in an amount of 15 mol% or more based on the whole amount of the feed and ethyl methyl carbonate in an amount of 12 mol% or more based on the whole amount of the feed,
the feed has a molar ratio dimethyl carbonate/(ethanol + ethyl methyl carbonate) of 0.1 to 2.0, and
a proportion of ethanol to ethyl methyl carbonate in the fraction A_{B} is 20 ppm by mass or less, and a proportion of methanol to ethyl methyl carbonate in the fraction A_{B} is 20 ppm by mass or less.

According to the present embodiment, ethyl methyl carbonate having low ethanol and methanol contents can be produced with high efficiency. More specifically, since the proportion of the alcohol content is reduced in the present embodiment, EMC can be produced with high efficiency in view of capable of reducing the reflux ratio of the distillation column or capable of reducing the amount of loss of EMC.

It has been surprisingly found that in the method for producing EMC according to the present embodiment, EMC can be highly purified with high efficiency by a high concentration of DMC in a solution fed to the distillation column. The mechanism of exhibiting such an effect is presumed that when the concentration of DMC in the solution to be fed is as high as the predetermined value or more as mentioned above, the formation of the azeotropic composition of EMC and EtOH at ordinary pressure can be eliminated by an azeotropic distillation effect.

For example, the method for producing EMC according to the present embodiment includes:
a reaction step of subjecting DMC and EtOH to a transesterification reaction in a reaction apparatus R to obtain a reaction composition,
distillation step A of feeding the reaction composition to a distillation column A as a feed containing DMC, EMC, and EtOH, and extracting a fraction A_{B} from the column bottom of the distillation column A,
distillation step B of feeding the fraction A_{B} to a distillation column B, and extracting a fraction B_{B} from which a low-boiling point substance having a lower boiling point than a boiling point of ethyl methyl carbonate has been removed, from a column bottom of the distillation column B, and
distillation step C of feeding the fraction B_{B} to a distillation column C, and extracting a fraction C_{L} from a column upper part of the distillation column C,
in the order presented.

In the method for producing EMC according to the present embodiment, any product obtained from any of the steps may be regarded as the target product. For example, a composition that contains EMC having a reduced content of alcohols can be obtained from the reaction composition through distillation step A. EMC having a purity of 99.99% by mass or more can be obtained from the reaction composition through distillation step A, distillation step B, and distillation step C. Note that, EMC having a purity of 99.99% by mass or more is used for applications such as battery electrolytes.

### <Reaction Step>

In the reaction step, DMC and EtOH are subjected to a transesterification reaction in the reaction apparatus R to obtain a reaction composition. The reaction composition obtained by the transesterification reaction may contain EMC, DMC, DEC, MeOH, and EtOH.

The raw material fed to the reaction apparatus R contains DMC and EtOH. Various components may be biomass-derived compounds. EtOH may be bioethanol or DMC obtained by using DMC biomethanol as the raw material. The raw material may contain an alkali metal compound as the catalyst. The feed of the raw material may be continuously carried out.

Examples of an alkali metal compound used as the catalyst include sodium hydroxide, potassium hydroxide, and an alkoxide of an alkali metal. Examples of the alkoxide of an alkali metal include lithium methoxide, lithium ethoxide, sodium methoxide, sodium methoxide, sodium ethoxide, potassium methoxide, and potassium ethoxide. Among these, the alkali metal compound is preferably a sodium-containing compound, more preferably sodium methoxide or sodium ethoxide, and further preferably sodium methoxide, because the high activity thereof enables economical operation with a low catalyst concentration.

The concentration of the alkali metal compound is preferably 1 ppm by mass to 30,000 ppm by mass, more preferably 10 ppm by mass to 1,000 ppm by mass, and further preferably 25 ppm by mass to 500 ppm by mass based on the total amount of the raw material.

### (Reaction Apparatus R)

Examples of the reaction apparatus R include a stirred reactor and a reaction distillation column. Since the catalyst or the reactant thereof is easily precipitated in the reaction step, the reaction apparatus R is preferably a stirred reactor.

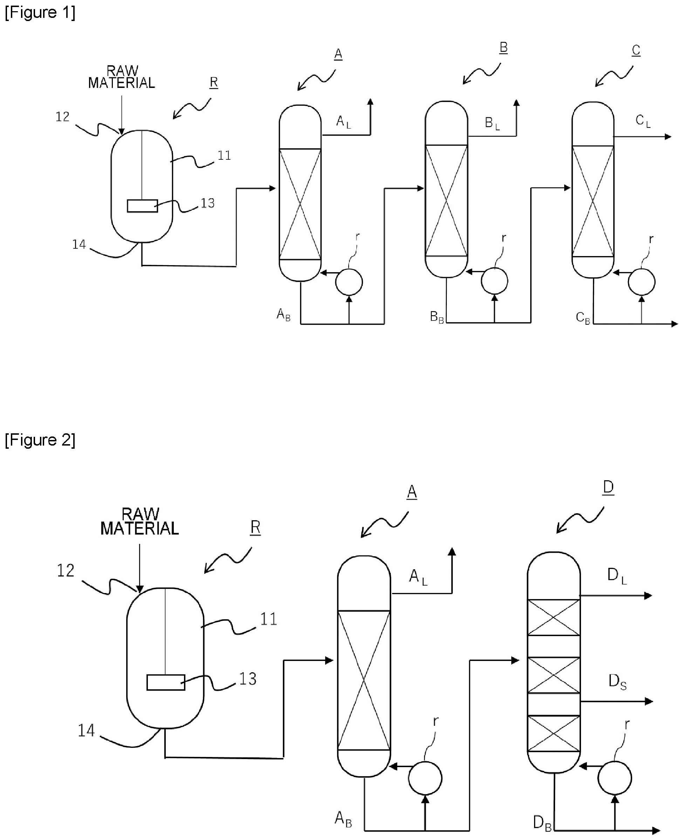

As shown in Figure 1, the reaction apparatus R has a reaction vessel 11. In the reaction apparatus R, a raw material feed part 12 is provided on the reaction vessel 11, and the raw material is fed. Here, the raw material is the aforementioned raw material, which contains DMC and EtOH and may contain a catalyst that contains an alkali metal compound. The reaction apparatus R has a stirring apparatus 13 in the reaction vessel 11. An extraction part 14 is provided in the lower part of the reaction vessel 11, and the reaction composition is extracted from the extraction part 14.

The reaction temperature in the reaction step is preferably 40°C to 150°C, more preferably 50°C to 130°C, and further preferably 60°C to 120°C.

The pressure in the reaction step may be, for example, ordinary pressure to 1000 kPaG.

When the raw material is continuously fed using a stirred reactor, the reaction composition is preferably extracted from the stirred reactor. The extraction of the reaction composition may be continuously carried out. The extraction rate of the reaction composition is preferably set such that the aforementioned reactor residence time is achieved.

The production method according to the present embodiment may include an acid addition step of adding an acidic substance having a pKa of 6.0 or less to the reaction composition after the reaction step. By providing the acid addition step, the alkali metal compound remained in the reaction composition can be deactivated.

The pKa of the acidic substance is preferably 0.0 to 5.8, more preferably 1.0 to 5.6, and further preferably 2.0 to 5.4.

Examples of the acidic substance include organic acids. Examples of the organic acid include carboxylic acids and sulfonic acids. Examples of the carboxylic acid include carboxylic acid having 2 to 30 carbon atoms, and more specific examples thereof include acetic acid, dichloroacetic acid, propionic acid, butanoic acid, hexanoic acid, and oleic acid. Among these acidic substances, at least one selected from the group consisting of carboxylic acids is preferable, and at least one selected from the group consisting of propanoic acid, butanoic acid, pentanoic acid, hexanoic acid, chloroacetic acid, dichloroacetic acid, trichloroacetic acid, and oleic acid is more preferable.

The amount of the acidic substance added in the acid addition step is preferably 2.0 to 15.0, more preferably 2.5 to 12.0, and further preferably 3.0 to 10.0 in terms of a molar ratio the acidic substance/the catalyst.

In the acid addition step, the acidic substance may be added into a stirred tank, or the reaction composition and the acidic substance may be joined together and then mixed using a static mixer.

The production method according to the present embodiment may have a solvent addition step of adding a solvent to the reaction composition, after the reaction step or after the acid addition step, and before distillation step A mentioned below. By adding a solvent and thereby dissolving the catalyst contained in the reaction composition, the production of the precipitate in distillation step A can be more significantly suppressed. The insoluble matter in the reaction composition is preferably dissolved by the solvent addition step. That is, the solvent is preferably added in such an amount that the insoluble matter contained in the reaction composition is dissolved.

Here, the solvent to be added preferably provides a solubility of 10% by mass or more at 30°C of the alkali metal compound as well as of the reaction product of the alkali metal compound and the acidic substance. By providing the above solubility, the catalyst contained in the reaction composition can further be dissolved, and the production of the precipitate in the distillation step can be more significantly suppressed.

The solubility in the solvent of the alkali metal compound as well as of the reaction product of the alkali metal compound and the acidic substance is more preferably 10% by mass or more, and further preferably 15% by mass or more at 30°C. The above solubility may be, for example, 50% by mass or less at 30°C.

Here, the boiling point of the solvent is preferably 136°C or more, more preferably 140°C to 400°C, and further preferably 150°C to 300°C under ordinary pressure.

Here, the boiling point of the solvent preferably has a higher boiling point than the boiling point of EMC, more preferably has a boiling point 10°C or more higher than the boiling point of EMC, and further preferably has a boiling point 20°C or more higher than the boiling point of EMC, under ordinary pressure.

The solvent added in the solvent addition step preferably contains a compound having at least one hydroxyl group, and more preferably contains a compound having at least two hydroxyl groups. Examples of the solvent include monoethylene glycol, diethylene glycol, triethylene glycol, 1-hexanol, 2-hexanol, 1-heptanol, and 2-heptanol. Among these, monoethylene glycol, diethylene glycol, or triethylene glycol is preferable.

The content of the aforementioned compound having at least one hydroxyl group is preferably 50% by mass to 100% by mass, more preferably 70% by mass to 100% by mass, and further preferably 90% by mass to 100% by mass based on the whole amount of the solvent.

The amount of the solvent added in the solvent addition step is preferably 0.1 parts by mass to 20 parts by mass, more preferably 0.5 parts by mass to 15 parts by mass, and further preferably 1.0 part by mass to 10 parts by mass per 100 parts by mass of the reaction composition, in view of more significantly suppressing the production of the precipitate in the distillation step and reducing an operation load on the distillation column.

In the solvent addition step, the solvent may be added into a stirred tank, or the reaction composition and the solvent may be joined together and then mixed using a static mixer.

### <Distillation Step A>

In distillation step A, a feed containing DMC, EMC, and EtOH is fed to the distillation column A, and the fraction A_{B} is extracted from the column bottom of the distillation column A. Here, the feed may be the reaction composition obtained in the aforementioned reaction step, or a mixture containing the reaction composition and recycle components such as other fractions.

The feed contains DMC in an amount of 15 mol% or more based on the whole amount of the feed and EMC in an amount of 12 mol% or more based on the whole amount of the feed.

When DMC is contained in the above amount, the formation of the azeotropic composition of EMC and EtOH can be eliminated by the azeotropic distillation effect with EtOH in the distillation column A, and EtOH can be removed by distillation even at ordinary pressure, so that EMC having low ethanol and methanol contents can be produced with high efficiency.

The proportion of DMC is preferably 15 mol% to 60 mol%, more preferably 20 mol% to 50 mol%, and further preferably 25 mol% to 40 mol% based on the whole amount of the feed, in view of producing EMC having low ethanol and methanol contents with higher efficiency. When the proportion of DMC is the lower limit value or more, the alcohols can be separated even by distillation at ordinary pressure due to the aforementioned azeotropic effect of DMC and EtOH. When the proportion of DMC is the upper limit value or less, the amount of DMC introduced into the distillation column can be reduced, so that EMC can be purified with higher efficiency.

The proportion of EMC is preferably 12 mol% to 50 mol%, more preferably 15 mol% to 40 mol%, and further preferably 17 mol% to 30 mol% based on the whole amount of the feed, in view of producing EMC having low ethanol and methanol contents with higher efficiency. When the proportion of EMC is the lower limit value or more, the amount of EMC introduced into the distillation column A can be sufficiently ensured, so that EMC can be purified with higher efficiency. When the proportion of EMC is the upper limit value or less, the alcohols can be separated even by distillation at ordinary pressure due to the aforementioned azeotropic effect of DMC and EtOH.

The feed has a molar ratio dimethyl carbonate/(ethanol + ethyl methyl carbonate) of 0.1 to 2.0. With the above ratio, the formation of the azeotropic composition of EMC and EtOH can be eliminated by the azeotropic distillation effect of DMC and EtOH in the distillation column A, and ethanol can be removed from EMC by distillation even at ordinary pressure, so that EMC having low ethanol and methanol contents can be produced with high efficiency. The molar ratio DMC/(EtOH + EMC) is preferably 0.3 to 1.6, more preferably 0.4 to 1.5, and further preferably 0.5 to 1.4.

The molar ratio DMC/EMC is preferably 0.8 to 2.5, more preferably 0.9 to 2.3, and further preferably 1.0 to 2.1, in view of eliminating the azeotropy of EMC and ethanol and producing EMC having low ethanol and methanol contents with higher efficiency.

The proportion of EtOH in the feed is preferably 5 mol% to 40 mol%, more preferably 7 mol% to 30 mol%, and further preferably 9 mol% to 25 mol% based on the whole amount of the feed, in view of producing EMC having low ethanol and methanol contents with higher efficiency.

The proportion of MeOH in the feed is preferably 5 mol% to 50 mol%, more preferably 10 mol% to 40 mol%, and further preferably 15 mol% to 35 mol% based on the whole amount of the feed, in view of producing EMC having low ethanol and methanol contents with higher efficiency.

The proportion of DEC in the feed is preferably 0.1 mol% to 20 mol%, more preferably 0.5 mol% to 15 mol%, and further preferably 1.0 mol% to 10 mol% based on the whole amount of the feed, in view of producing EMC having low ethanol and methanol contents with higher efficiency.

In the aforementioned proportions, the whole amount of the feed is the proportion to the total amount of MeOH, EtOH, DMC, EMC, and DEC by mole. When the solvent added in the aforementioned solvent addition step is a solvent other than MeOH, EtOH, DMC, EMC, and DEC, the solvent is eliminated from the whole amount of the feed as used herein.

Various components contained in the feed may be biomass-derived compounds. Examples of the biomass-derived compound include bioethanol, biomethanol, as well as EMC, DMC, and DEC obtained by using at least one selected from the group consisting of bioethanol and biomethanol as the raw material.

With respect to the method for feeding the feed to the distillation column A, the reaction composition obtained in the reaction step may be fed as it is, or DMC may be additionally fed with the reaction composition. The method for feeding at least a part of DMC in the feed to the distillation column A is preferably a single addition of a liquid with a pump.

As the distillation column A, for example, a continuous distillation column can be used without particular limitation. The distillation column A typically includes a reboiler for heating the column bottom part.

The distillation column A preferably includes a tray and/or filling as the internal. The internal means a part in the distillation column where gas and liquid are actually brought into contact with each other. Examples of the tray include foam trays, porous plate trays, ripple trays, Ballast trays, valve trays, countercurrent trays, uniflux trays, SUPERFRAC trays, Max-Frac trays, dual flow trays, grid plate trays, turbogrid plate trays, and kittel trays. Examples of the filling include random packings such as Raschig rings, Lessing rings, Pall rings, Berl saddles, Intalox saddles, Dixon packings, McMahon packings, and Heli Pack, and regular packings such as MellaPak, Gempak, Techno Pak, FLEXIPAC, Sulzer packings, Goodloe packings, and Glitch Grid.

Examples of the numerical value that represents the separation capacity of the distillation column include the number of theoretical plates. The number of theoretical plates of the distillation column is preferably 13 or more, preferably 22 or more, more preferably 25 or more, and further preferably 30 or more. By using a distillation column having the number of theoretical plates in such a range, EMC having high purity can be purified with high efficiency. The internal of the distillation column may be the tray or the filling. The material of the internal is not particularly limited, and may be porcelain or metal.

The feed may be introduced from the column intermediate part of the distillation column A. The term "column intermediate part" means a part of the distillation column excluding the column top and the column bottom. When the distillation column A includes the internal, the column intermediate part is preferably located between the positions at 1/2 and 5/6, from the bottom of the filling, of the entire length of the internal filled.

The feed temperature of the feed is preferably 25°C to 100°C, more preferably 30°C to 90°C, and further preferably 35°C to 80°C.

The column bottom temperature of the distillation column A is preferably 80°C to 250°C, more preferably 90°C to 200°C, further preferably 100°C to 150°C, and further more preferably 105°C to 120°C. When the column bottom temperature of the distillation column A is in the above range, EMC having low ethanol and methanol contents can be produced with high energy efficiency.

The column top pressure in the distillation column A is preferably 0 kPaG to 50 kPaG, more preferably 0 kPaG to 30 kPaG, and further preferably 0 kPaG to 20 kPaG. In the present embodiment, when DMC is contained in an amount in a predetermined range in the feed, the alcohols can be removed by distillation even under at a column top pressure in the above range, and EMC having low ethanol and methanol contents can be produced with high energy efficiency.

The column bottom pressure in the distillation column A is preferably 0.5 kPaG to 80 kPaG, more preferably 1 kPaG to 60 kPaG, and further preferably 2 kPaG to 40 kPaG.

The reflux ratio of the distillation column A is preferably 0.3 to 20, more preferably 0.5 to 15, and further preferably 0.7 to 5.

In the distillation column A, the feed is distilled, the fraction A_{B} is extracted from the column bottom, and the fraction A_{L} is extracted from the upper part. The fraction A_{L} may be extracted from the column top.

The proportion of ethanol to ethyl methyl carbonate in the fraction A_{B} obtained through distillation step A is 20 ppm by mass or less, and the proportion of methanol to ethyl methyl carbonate in the fraction A_{B} is 20 ppm by mass or less. In distillation step A, the conditions of distillation are adjusted so as to achieve the above contents of the alcohols. The conditions of distillation can be adjusted so as to achieve the above contents of ethanol and methanol by adjusting the column bottom temperature and others, when DMC is contained in a predetermined range in the feed. The proportion of ethanol to EMC in the fraction A_{B} obtained through distillation step A is preferably 17 ppm by mass or less, more preferably 15 ppm by mass or less, and further preferably 10 ppm by mass or less. The proportion of methanol to EMC in the fraction A_{B} obtained through distillation step A is preferably 17 ppm by mass or less, more preferably 15 ppm by mass or less, and further preferably 10 ppm by mass or less.

In view of suppressing the loss of EMC and reducing the amount of energy consumed in distillation step A, the EMC content in the fraction A_{L} is preferably 0.5% by mass to 10% by mass, more preferably 0.7% by mass to 8% by mass, and further preferably 1.0% by mass to 7% by mass. In distillation step A, distillation is preferably carried out by adjusting the temperature and pressure such that the EMC content in the fraction A_{L} falls within the above range.

Similarly, in view of suppressing the loss of EMC and reducing the amount of energy consumed in distillation step A, the DMC content in the fraction A_{B} is preferably 0.001% by mass to 5.0% by mass, more preferably 0.0015% by mass to 4.0% by mass, and further preferably 0.002% by mass to 3.5% by mass. In distillation step A, distillation is preferably carried out by adjusting the temperature and pressure such that the EMC content in the fraction A_{L} falls within the above range.

According to distillation step A above, EMC having low ethanol and methanol contents can be produced with high efficiency. Consequently, by further distilling the obtained fraction, EMC having high purity can be obtained with high efficiency.

Examples of the method for producing EMC having higher purity include (1), (2), or (3) below:
(1) the method further including: distillation step B of feeding the fraction A_{B} to a distillation column B, and extracting a fraction B_{B} from which a low-boiling point substance having a lower boiling point than a boiling point of ethyl methyl carbonate has been removed, from a column bottom of the distillation column B; and distillation step C of feeding the fraction B_{B} to a distillation column C, and extracting a fraction C_{L} from a column upper part of the distillation column C, wherein ethyl methyl carbonate purity in the fraction C_{L} is 99.99% by mass or more;
(2) the method further including: distillation step D of feeding the fraction A_{B} to a distillation column D, and extracting a fraction D_{B} from a column bottom of the distillation column D, a fraction D_{L} from a column top, and a fraction D_{S} from the intermediate part of the distillation column D, wherein ethyl methyl carbonate purity in the fraction D_{S} is 99.99% by mass or more; or
(3) the method further including: distillation step B of feeding the fraction A_{B} to a distillation column B, and extracting a fraction B_{B} from which a low-boiling point substance having a lower boiling point than a boiling point of ethyl methyl carbonate has been removed, from a column bottom of the distillation column B; and distillation step D of feeding the fraction B_{B} to a distillation column D, and extracting a fraction D_{B} from the column bottom of the distillation column D, a fraction D_{L} from the column top, and a fraction D_{S} from the intermediate part of the distillation column D, wherein ethyl methyl carbonate purity in the fraction D_{S} is 99.99% by mass or more.

### <Distillation Step B>

The method for producing EMC according to the present embodiment may have distillation step B of feeding the fraction A_{B} to the distillation column B, and extracting the fraction B_{B} from which a low-boiling point substance having a lower boiling point than the boiling point of ethyl methyl carbonate has been removed, from the column bottom of the distillation column B. In distillation step B, the low-boiling point substance having a lower boiling point than the boiling point of ethyl methyl carbonate, such as DMC, contained in the fraction A_{B} is removed. With respect to the term "the low-boiling point substance has been removed" as used herein, the low-boiling point substance is not required to have been completely removed, and the content of the low-boiling point substance in the fraction B_{B} is only required to be lower than the content of the low-boiling point substance in the fraction A_{B}.

As the distillation column B used in distillation step B, the distillation column described as the example of the distillation column A may be used. The number of theoretical plates of the distillation column B is preferably 30 or more, more preferably 35 or more, and further preferably 40 or more. By using a distillation column having the number of theoretical plates in such a range, impurities other than ethanol and/or methanol (e.g., DMC) can be further removed, and the EMC purity can be increased. The internal of the distillation column may be the tray or the filling. The material of the internal is not particularly limited, and may be porcelain, metal, or the like.

The fraction A_{B} may be introduced from the column intermediate part of the distillation column B. When the distillation column B includes the internal, the column intermediate part is preferably located between the position at 1/4 and 5/6, from the bottom of the filling, of the entire length of the internal filled.

The column bottom temperature of the distillation column B is preferably 80°C to 250°C, more preferably 90°C to 200°C, further preferably 100°C to 150°C, and further more preferably 105°C to 120°C.

The column top pressure in the distillation column B is preferably 0 kPaG to 50 kPaG, more preferably 0 kPaG to 30 kPaG, and further preferably 0 kPaG to 20 kPaG.

The column bottom pressure in the distillation column B is preferably 0.5 kPaG to 80 kPaG, more preferably 1 kPaG to 60 kPaG, and further preferably 2 kPaG to 40 kPaG.

The reflux ratio of the distillation column B is preferably 5 to 1,500, more preferably 10 to 1,400, and further preferably 15 to 1,350.

The purity of EMC in the fraction B_{B} is preferably 50% by mass to 90% by mass, more preferably 60% by mass to 90% by mass, and further preferably 70% by mass to 90% by mass.

The DMC content in the fraction B_{B} is preferably 50 ppm by mass or less, more preferably 20 ppm by mass or less, further preferably 17 ppm by mass or less, and further more preferably 15 ppm by mass or less.

The DMC content in the fraction B_{L} is preferably 5.0% by mass to 98% by mass, more preferably 10% by mass to 98% by mass, and further preferably 13% by mass to 98% by mass. When the DMC content in the fraction B_{L} is in such a range, impurities other than ethanol and/or methanol (e.g., DMC) can be reduced, and the EMC purity can be increased.

### <Distillation Step C>

The method for producing EMC according to the present embodiment may include distillation step C of feeding the fraction B_{B} to a distillation column C, and extracting a fraction C_{L} from the column upper part of the distillation column C. In distillation step C, the component having a higher boiling point than EMC, such as DEC, contained in the fraction B_{B} is removed.

As the distillation column C used in distillation step C, the distillation column described as the example of the distillation column A may be used. The number of theoretical plates of the distillation column is preferably 30 or more, more preferably 35 or more, and further preferably 40 or more. Impurities other than ethanol and/or methanol (e.g., DMC) can be reduced, and the EMC purity can be increased. The internal of the distillation column may be the tray or the filling. The material of the internal is not particularly limited, and may be porcelain, metal, or the like.

The fraction B_{B} may be introduced from the column intermediate part of the distillation column C. When the distillation column C includes the internal, the column intermediate part is preferably located between the position at 1/4 and 5/6, from the bottom of the filling, of the entire length of the internal filled. The extraction position of the fraction C_{L} is preferably positioned above the feed position of the fraction B_{B} into the distillation column C. Both the feed part and the extraction part may be provided on the column intermediate part, and in this case, the extraction part is only required to be arranged relatively above the feed part. The column upper part from which the fraction C_{L} is extracted means a position between the position at 5/6 from the bottom of the filling and the column top. The extraction position is preferably in the upper part. Namely, the fraction C_{L} may be extracted from the column top, or may be side cut at a position at 5/6 or more above the bottom.

The column bottom temperature of the distillation column C is preferably 80°C to 250°C, more preferably 90°C to 200°C, further preferably 100°C to 180°C, and further more preferably 105°C to 150°C.

The column top pressure in the distillation column C is preferably 0 kPaG to 50 kPaG, more preferably 0 kPaG to 30 kPaG, and further preferably 0 kPaG to 20 kPaG.

The column bottom pressure in the distillation column C is preferably 0.5 kPaG to 80 kPaG, more preferably 1 kPaG to 60 kPaG, and further preferably 2 kPaG to 40 kPaG.

The reflux ratio of the distillation column C is preferably 0.8 to 20, more preferably 1.2 to 15, and further preferably 1.5 to 10.

The EMC content in the fraction C_{B} is preferably 0.1% by mass to 5.0% by mass, more preferably 0.2% by mass to 4.0% by mass, and further preferably 0.25% by mass to 3.5% by mass. When the EMC content in the fraction C_{B} is in such a range, EMC having high purity can be purified with high efficiency. The DEC content in the fraction C_{L} is preferably 1 ppm by mass to 50 ppm by mass, more preferably 1 ppm by mass to 45 ppm by mass, and further preferably 1 ppm by mass to 40 ppm by mass.

The fraction C_{L} may be set to EMC as the final target.

### <Distillation Step D>

The method for producing EMC according to the present embodiment may include distillation step D of feeding the fraction A_{B} or the fraction B_{B} to the distillation column D, and extracting a fraction D_{B} from the column bottom of the distillation column D, a fraction D_{L} from the column top, and a fraction D_{S} from the intermediate part of the distillation column D. For example, by providing distillation step D instead of distillation steps B and C, the final product may be extracted from the intermediate part (side cut) in distillation step D to separate the final product from the low-boiling substance and the high-boiling substance.

That is, in distillation step D, the component having a lower boiling point than that of EMC, such as DMC, contained in the fraction A_{B} is removed from the fraction D_{L}, and the component having a higher boiling point than that of EMC, such as DEC, is removed from the fraction D_{B}.

As the distillation column D used in distillation step D, the same distillation column as the example of the distillation column A may be used. The column bottom temperature of the distillation column D is preferably 80°C to 250°C, more preferably 90°C to 200°C, further preferably 100°C to 180°C, and further more preferably 105°C to 150°C.

The column top pressure in the distillation column D is preferably 0 kPaG to 50 kPaG, more preferably 0 kPaG to 30 kPaG, and further preferably 0 kPaG to 20 kPaG.

The column bottom pressure in the distillation column D is preferably 0.5 kPaG to 80 kPaG, more preferably 1 kPaG to 60 kPaG, and further preferably 2 kPaG to 40 kPaG.

The reflux ratio of the distillation column D is preferably 5 to 1,500, more preferably 10 to 1,400, and further preferably 15 to 1,350.

The EMC content in the fraction D_{B} is preferably 0.1% by mass to 5.0% by mass, more preferably 0.2% by mass to 4.0% by mass, and further preferably 0.25% by mass to 3.5% by mass. When the EMC content in the fraction D_{B} is in such a range, EMC having high purity can be purified with high efficiency.

The DMC content in the fraction D_{L} is preferably 1.5% by mass to 20% by mass, more preferably 1.0% by mass to 30% by mass, and further preferably 2.0% by mass to 15% by mass.

EMC having high purity can be purified with high efficiency.

The method for producing EMC according to the present embodiment may further include a step of distilling the fraction C_{B} or fraction D_{B} extracted from the column bottom of the distillation column C or distillation column D to obtain diethyl carbonate having a purity of 99.99% by mass or more. Examples of the above step include steps of purifying diethyl carbonate, such as distillation step E or distillation step F.

### <Distillation Step E>

The method for producing EMC according to the present embodiment may include distillation step E of feeding the fraction C_{B} or fraction D_{B} to a distillation column E, and extracting a fraction E_{B} from which the low-boiling point substance having a lower boiling point than the boiling point of diethyl carbonate has been removed, from the column bottom of the distillation column E. In the distillation step E, the low-boiling point substance having a lower boiling point than the boiling point of diethyl carbonate, such as EMC, contained in the fraction C_{B} or fraction D_{B} is removed. With respect to the phrase "the low-boiling point substance has been removed" as used herein, the low-boiling point substance is not required to have been completely removed, and the content of the low-boiling point substance in the fraction E_{B} is only required to be lower than the content of the low-boiling point substance in the fraction C_{B} or fraction D_{B}.

As the distillation column E used in the distillation step E, the distillation column described as the example of the distillation column A may be used. The distillation column preferably has 15 or more, more preferably 20 or more, and further preferably 25 or more theoretical plates as the separation capacity of the distillation column, so that the low-boiling point substance having a lower boiling point than the boiling point of diethyl carbonate (e.g., EMC) can be further removed to increase the DEC purity. The internal may be the tray or the filling, as long as the distillation column has the above capacity. In addition, the material of the internal is not particularly limited to porcelain, metal, or the like.

The fraction C_{B} or the fraction D_{B} may be introduced from the column intermediate part of the distillation column E. When the distillation column E includes the internal, the column intermediate part is preferably located between the position at 1/4 and 5/6, from the bottom of the filling, of the entire length of the internal filled.

The column bottom temperature of the distillation column E is preferably 90°C to 250°C, more preferably 100°C to 200°C, further preferably 105°C to 150°C, and further more preferably 110°C to 140°C.

The column top pressure in the distillation column E is preferably 0 kPaG to 50 kPaG, more preferably 0 kPaG to 30 kPaG, and further preferably 0 kPaG to 20 kPaG.

The column bottom pressure in the distillation column E is preferably 0.5 kPaG to 80 kPaG, more preferably 1 kPaG to 60 kPaG, and further preferably 2 kPaG to 40 kPaG.

The reflux ratio of the distillation column E is preferably 5 to 2,000, more preferably 7.5 to 1,800, and further preferably 9.0 to 1,600.

The purity of DEC in the fraction E_{B} is preferably 99% by mass to 99.9995% by mass, more preferably 99.9% by mass to 99.999% by mass, and further preferably 99.99% by mass to 99.998% by mass.

The EMC content in the fraction E_{B} is preferably 50 ppm by mass or less, more preferably 40 ppm by mass or less, more preferably 30 ppm by mass or less, and further preferably 15 ppm by mass or less.

The DEC content in the fraction E_{L} is preferably 0.001% by mass to 90% by mass, more preferably 0.002% by mass to 80% by mass, and further preferably 0.01% by mass to 70% by mass. In such a range, the low-boiling point substance having a lower boiling point than the boiling point of diethyl carbonate (e.g., EMC) can be reduced, and the DEC purity can be increased.

### <Distillation Step F>

The method for producing EMC according to the present embodiment may include distillation step F of feeding the fraction C_{B} or the fraction D_{B} to a distillation column F, and extracting the fraction F_{B} from the column bottom of the distillation column F, the fraction F_{L} from the column top, and the fraction F_{S} from the intermediate part of the distillation column F. For example, by providing distillation step F instead of distillation step E, the final product may be extracted from the intermediate part (side cut) in distillation step F to separate the final product from the low-boiling substance and the high-boiling substance.

That is, in distillation step F, the component having a lower boiling point than that of DEC, such as EMC, contained in the fraction C_{B} or the fraction D_{B} is removed from the fraction F_{L}, and the component having a higher boiling point than that of DEC is removed from F_{B}. As the distillation column F used in distillation step F, the distillation column described as the example of the distillation column A may be used.

The column bottom temperature of the distillation column F is preferably 90°C to 250°C, more preferably 100°C to 200°C, further preferably 105°C to 150°C, and further more preferably 110°C to 140°C.

The column top pressure in the distillation column F is preferably 0 kPaG to 50 kPaG, more preferably 0 kPaG to 30 kPaG, and further preferably 0 kPaG to 20 kPaG.

The column bottom pressure in the distillation column F is preferably 0.5 kPaG to 80 kPaG, more preferably 1 kPaG to 60 kPaG, and further preferably 2 kPaG to 40 kPaG.

The reflux ratio of the distillation column F is preferably 5 to 2,000, more preferably 7.5 to 1,800, and further preferably 9.0 to 1,600.

The DEC content in the fraction F_{B} is preferably 30% by mass to 99% by mass, more preferably 40% by mass to 98% by mass, and further preferably 50% by mass to 95% by mass. In such a range, DEC having high purity can be purified with high efficiency.

The DEC content in the fraction F_{L} is preferably 0.001% by mass to 90% by mass, more preferably 0.002% by mass to 80% by mass, and further preferably 0.01% by mass to 70% by mass.

The purity of EMC obtained by the production method according to the present embodiment is preferably 99% by mass or more, more preferably 99.9% by mass or more, and further preferably 99.99% by mass or more.

The content of EtOH in the EMC final product obtained by the production method according to the present embodiment is preferably 20 ppm by mass or less, more preferably 17 ppm by mass or less, and further preferably 15 ppm by mass or less.

The content of MeOH in the EMC final product obtained by the production method according to the present embodiment is preferably 20 ppm by mass or less, more preferably 17 ppm by mass or less, and further preferably 15 ppm by mass or less.

The content of DMC in the EMC final product obtained by the production method according to the present embodiment is preferably 20 ppm by mass or less, more preferably 17 ppm by mass or less, and further preferably 15 ppm by mass or less.

The purity in the EMC final product and the analysis of each main component are measured by gas chromatography. The analysis by gas chromatography is carried out in accordance with HG/T 5158-2017.

Hereinafter, a production equipment used in the method for producing EMC according to the present embodiment, and the combination of respective steps in the case of using the production equipment will be described.

Figure 1 is a schematic diagram of the production equipment used in the method for producing EMC according to the present embodiment. The production equipment may include the reaction apparatus R, the distillation column A, the distillation column B, and the distillation column C. In the case of using the above production equipment, the method for producing EMC according to the present embodiment includes reaction step R, distillation step A, distillation step B, and distillation step C in the order presented.

Figure 2 is a schematic diagram of the production equipment used in the method for producing EMC according to the present embodiment. The production equipment may include the reaction apparatus R, the distillation column A, and the distillation column D. In the case of using the above production equipment, the method for producing EMC according to the present embodiment includes reaction step R, distillation step A, and distillation step D in the order presented. Since both the high-boiling point component and the low-boiling point component contained in the fraction A_{B} can be simultaneously removed in the distillation step D by the above production method, the number of distillation steps and the number of distillation columns in the equipment can be reduced.

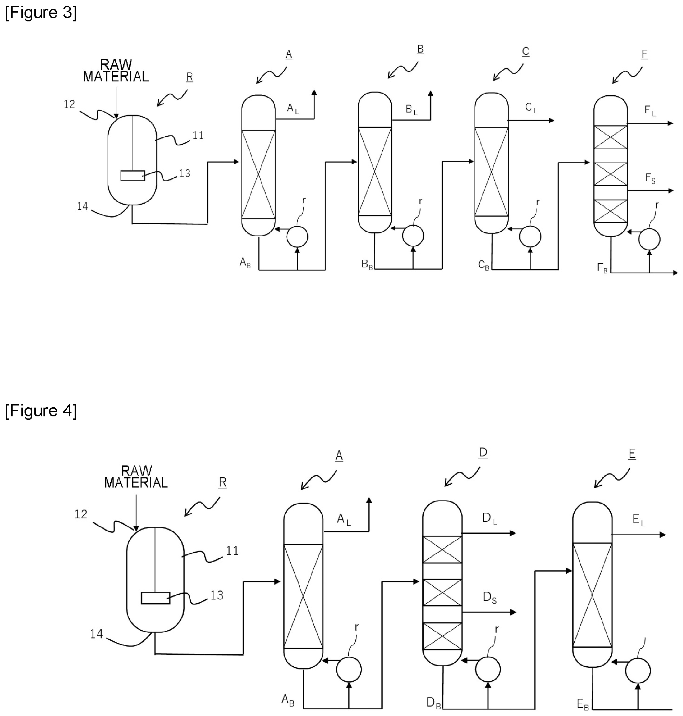

Figure 3 is a schematic diagram of the production equipment used in the method for producing EMC according to the present embodiment. The production equipment according to the present embodiment may include the reaction apparatus R, the distillation column A, the distillation column B, the distillation column C, and the distillation column F. In the case of using the above production equipment, the method for producing EMC according to the present embodiment includes reaction step R, distillation step A, distillation step B, distillation step C, and distillation step F in the order presented. By including distillation step F, not only EMC, but also DEC can be recovered as the final product.

Figure 4 is a schematic diagram of the production equipment used in the method for producing EMC according to the present embodiment. The production equipment according to the present embodiment may include the reaction apparatus R, the distillation column A, the distillation column D, and the distillation column E. In the case of using the above production equipment, the method for producing EMC according to the present embodiment includes reaction step R, distillation step A, distillation step D, and distillation step E in the order presented. By including distillation step E, not only EMC, but also DEC can be recovered as the final product.

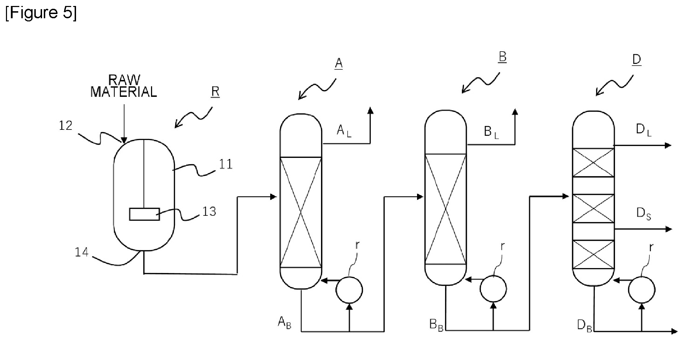

Figure 5 is a schematic diagram of the production equipment used in the method for producing EMC according to the present embodiment. The production equipment may include the reaction apparatus R, the distillation column A, the distillation column B, and the distillation column D. In the case of using the above production equipment, the method for producing EMC according to the present embodiment includes reaction step R, distillation step A, distillation step B, and distillation step D in the order presented. Since both the high-boiling point component and the low-boiling point component contained in the fraction B_{B} can be simultaneously removed by the above production method in the distillation step D, EMC having higher purity can be obtained.

### Examples

Hereinafter, the present embodiment will be described in more detail by way of Examples, but the present embodiment is not limited to the following Examples.

### [Example 1]

The method for producing ethyl methyl carbonate was conducted using the apparatus shown in Figure 1. DMC, EtOH, and a 3.2% by mass (1.9 mol%) solution of sodium methoxide in methanol were respectively fed to a stirred tank reactor having a capacity of 1.5 m3 at a flow rate of 475.2 kg/h (5.28 kmol/h), 210.7 kg/h (4.58 kmol/h), and 4.3 kg/h (0.13 kmol/h), and reacted under the conditions of 50°C and 103 kPaG, and the reactant was extracted from the stirred tank reactor at 690 kg/h (9.99 kmol/h) to obtain a reaction composition containing EMC.

The reaction composition was filtered, then fed to a stirred tank holding 20 kg of a solid adsorbent, stirred for 1 hour, then filtered, and fed to the distillation column A, followed by distillation as mentioned below.

In a distillation column made of carbon steel and having 60 porous plate trays, the purification of EMC was carried out as follows. The separation capacity of the distillation column was 39 theoretical plates.

MeOH, EtOH, DMC, EMC, and DEC were respectively fed at 2.85 kmol/h, 1.86 kmol/h, 2.93 kmol/h, 1.98 kmol/h, and 0.37 kmol/h onto the 46th tray from the bottom of the distillation column as a liquid of 50°C. In the feed liquid, the DMC concentration was 29.3 mol%, and the molar ratio of DMC to the total amount of EtOH and EMC was 0.76. The distillation column A was continuously operated at a column top pressure of 0.0 kPaG, a column bottom pressure of 3.1 kPaG, a column bottom temperature of 109.2°C, and a reflux ratio of 1.7.

A liquid was continuously extracted with a pump at 2.36 kmol/h from the column bottom part of the distillation column A. In the liquid extracted from the column bottom part (fraction A_{B}), the flow rate was 0.000000 mol/h for EtOH, 0.01 kmol/h for DMC, 1.98 kmol/h for EMC, and 0.37 kmol/h for DEC, and the total amount of ethanol and methanol to the EMC mass in the column bottom liquid was 0 ppm by mass, so that 20 ppm by mass or less was achieved. In addition, a liquid was continuously extracted with a pump at 7.63 kmol/h from the column top part of the distillation column A. In the liquid extracted from the column top part (fraction A_{L}), the flow rate was 2.85 kmol/h for MeOH, 1.86 kmol/h for EtOH, 2.92 kmol/h for DMC, and 0.001 kmol/h for EMC.

The extraction liquid from the column bottom part of the distillation column A (fraction A_{B}) was fed to the distillation column B and distilled as mentioned below.

The distillation column B was a distillation column made of SUS304 and having 27 m of a regular packing, MellaPak752Y, and the purification of EMC was carried out as follows. The separation capacity of the distillation column was 48 theoretical plates.

The extraction liquid from the column bottom part of the distillation column A (fraction A_{B}) was fed to the position 13.0 m away from the lowest part of the filling in the distillation column B as a liquid of 107°C. The distillation column B was operated at a column top pressure of -40.0 kPaG, a column bottom pressure of -37.9 kPaG, a column bottom temperature of 93.7°C, and a reflux ratio of 1,100.

A liquid was continuously extracted with a pump at 2.35 kmol/h from the column bottom part of the distillation column B. In the liquid extracted from the column bottom part (fraction B_{B}), the flow rate was 0.0001 kmol/h for DMC, 1.98 kmol/h for EMC, and 0.37 kmol/h for DEC, and the DMC content in the column bottom liquid was 25 ppm by mass, so that 50 ppm by mass or less was achieved. In addition, a liquid was continuously extracted with a pump at 0.01 kmol/h from the column top part of the distillation column B. In the liquid extracted from the column top part (fraction B_{L}), the flow rate was 0.01 kmol/h for DMC and 0.002 kmol/h for EMC.

The extraction liquid from the column bottom part of the distillation column B (fraction B_{B}) was fed to the distillation column C and distilled as mentioned below.

The distillation column C was a distillation column made of SUS316 and having 49 m of a regular packing, MellaPak250Y, and the purification of EMC was carried out as follows. The separation capacity of the distillation column was 47 theoretical plates.

The extraction liquid from the column bottom part of the distillation column B (fraction B_{B}) was fed to the position 14.0 m away from the lowest part of the filling in the distillation column as a liquid of 93°C. The distillation column was operated at a column top pressure of 0.0 kPaG, a column bottom pressure of 2.1 kPaG, a column bottom temperature of 127.5°C, and a reflux ratio of 2.0.

A liquid was continuously extracted with a pump at 0.43 kmol/h from the column bottom part of the distillation column C. In the liquid extracted from the column bottom part (fraction C_{B}), the flow rate was 0.06 kmol/h for EMC and 0.37 kmol/h for DEC, and the EMC content in the column bottom liquid was 11.9% by mass. In addition, a liquid was continuously extracted with a pump at 1.92 kmol/h from the column top part of the distillation column. In the liquid extracted from the column top part (fraction C_{L}), the flow rate was 0.00007 kmol/h for DMC, 1.92 kmol/h for EMC, and 0.00008 kmol/h for DEC, so that the purification of EMC having a purity of 99.99% by mass or more was achieved.

### [Example 2]

The method for producing ethyl methyl carbonate was conducted using the apparatus shown in Figure 2. Using a distillation column made of SUS304 and having 19 m of a regular packing, MellaPak752Y as the distillation column A, the purification of EMC was carried out as follows. The separation capacity of the distillation column was 47 theoretical plates.

MeOH, EtOH, DMC, EMC, and DEC were respectively fed at 3.33 kmol/h, 1.74 kmol/h, 3.67 kmol/h, 2.08 kmol/h, and 0.26 kmol/h to the position 12.7 m away from the lowest part of the filling in the distillation column as a liquid of 50°C. Similarly, DMC was fed at 0.408 kmol/h to the position 12.7 m away from the lowest part of the filling in the distillation column as a liquid of 50°C. In the feed liquid, the DMC concentration was 4.3 mol%, and the molar ratio of DMC to the total amount of EtOH and EMC was 0.11. The distillation column A was continuously operated at a column top pressure of 2.3 kPaG, a column bottom pressure of 3.8 kPaG, a column bottom temperature of 107.8°C, and at a reflux ratio of 1.0.

A liquid was continuously extracted with a pump at 2.38 kmol/h from the column bottom part of the distillation column A. In the liquid extracted from the column bottom part (fraction A_{B}), the flow rate was 0.00000 kmol/h for EtOH, 0.06 kmol/h for DMC, 2.06 kmol/h for EMC, and 0.26 kmol/h for DEC, and the total amount of ethanol and methanol to the EMC mass in the column bottom liquid was 0 ppm by mass, so that 20 ppm by mass or less was achieved. In addition, a liquid (fraction A_{L}) was continuously extracted with a pump at 8.69 kmol/h from the column top part of the distillation column A. In the liquid extracted from the column top part, the flow rate was 3.33 kmol/h for MeOH, 1.74 kmol/h for EtOH, 3.61 kmol/h for DMC, and 0.018 kmol/h for EMC.

The extraction liquid from the column bottom part of the distillation column A (fraction A_{B}) was fed to the distillation column D and distilled as mentioned below.

The distillation column D was a distillation column made of SUS304 and having 47 m of a regular packing, MellaPak752Y, and the purification of EMC was carried out as follows. The separation capacity of the distillation column was 47 theoretical plates.

Gas was continuously extracted at 1.92 kmol/h from the position 26 m away from the lowest part of the filling in the distillation column D, condensed with a condenser, and then continuously extracted with a pump as the side cut. In the liquid extracted from the side cut (fraction D_{S}), the flow rate was 0.0001 kmol/h for DMC, 1.92 kmol/h for EMC, and 0.00005 kmol/h for DEC, so that the purification of EMC having a purity of 99.99% by mass or more was achieved. In addition, a liquid (fraction D_{B}) was continuously extracted with a pump at 0.39 kmol/h from the column bottom part of the distillation column D. In the liquid extracted from the column bottom part (fraction D_{B}), the flow rate was 0.13 kmol/h for EMC and 0.26 kmol/h for DEC. Further, a liquid (fraction D_{L}) was continuously extracted with a pump at 0.062 kmol/h from the column top part of the distillation column D. In the liquid (fraction D_{L}) extracted from the column top part, the flow rate was 0.06 kmol/h for DMC and 0.002 kmol/h for EMC.

### [Example 3]

The method for producing ethyl methyl carbonate was conducted using the apparatus shown in Figure 1. The distillation column A was a distillation column made of carbon steel and having 20 porous plate trays, and the purification of EMC was carried out as follows. The separation capacity of the distillation column was 13 theoretical plates.

MeOH, EtOH, DMC, EMC, and DEC were respectively fed at 2.85 kmol/h, 1.86 kmol/h, 2.93 kmol/h, 1.98 kmol/h, and 0.37 kmol/h onto the 8th tray from the bottom of the distillation column A as a liquid of 50°C. In the feed liquid, the DMC concentration was 29.3 mol%, the molar ratio of DMC to the total amount of EtOH and EMC was 0.76. The distillation column A was continuously operated at a column top pressure of 0.0 kPaG, a column bottom pressure of 1.0 kPaG, a column bottom temperature of 105.7°C, and a reflux ratio of 2.0.

A liquid (fraction A_{B}) was continuously extracted with a pump at 2.60 kmol/h from the column bottom part of the distillation column A. In the liquid extracted from the column bottom part (fraction A_{B}), the flow rate was 0.000003 mol/h for EtOH, 0.34 kmol/h for DMC, 1.89 kmol/h for EMC, and 0.37 kmol/h for DEC, and the total amount of ethanol and methanol to the EMC mass in the column bottom liquid was 1 ppm by mass, so that 20 ppm by mass or less was achieved. In addition, a liquid (fraction A_{L}) was continuously extracted with a pump at 7.39 kmol/h from the column top part of the distillation column. In the liquid extracted from the column top part (fraction A_{L}), the flow rate was 2.85 kmol/h for MeOH, 1.86 kmol/h for EtOH, 2.59 kmol/h for DMC, and 0.093 kmol/h for EMC.

The extraction liquid from the column bottom part of the distillation column A (fraction A_{B}) was fed to the distillation column B and distilled as mentioned below.

The distillation column B was a distillation column made of SUS304 and having 27 m of a regular packing, MellaPak752Y, and the purification of EMC was carried out as follows. The separation capacity of the distillation column was 48 theoretical plates.

The extraction liquid from the column bottom part of the distillation column A (fraction A_{B}) was fed to the position 13.0 m away from the lowest part of the filling in the distillation column as a liquid of 107°C. The distillation column B was operated at a column top pressure of -40.0 kPaG, a column bottom pressure of -37.9 kPaG, a column bottom temperature of 93.7°C, and a reflux ratio of 20.

A liquid (fraction B_{B}) was continuously extracted with a pump at 2.26 kmol/h from the column bottom part of the distillation column B. In the liquid extracted from the column bottom part (fraction B_{B}), the flow rate was 0.0001 kmol/h for DMC, 1.89 kmol/h for EMC, and 0.37 kmol/h for DEC, the DMC content in the column bottom liquid was 47 ppm by mass, so that 50 ppm by mass or less was achieved. In addition, a liquid (fraction B_{L}) was continuously extracted with a pump at 0.34 kmol/h from the column top part of the distillation column B. In the liquid extracted from the column top part (fraction B_{L}), the flow rate was 0.34 kmol/h for DMC and 0.001 kmol/h for EMC.

The extraction liquid from the column bottom part of the distillation column B (fraction B_{B}) was fed to the distillation column C and distilled as mentioned below.

The distillation column C was a distillation column made of SUS316 and having 49 m of a regular packing, MellaPak250Y, and the purification of EMC was carried out as follows. The separation capacity of the distillation column was 47 theoretical plates.

The extraction liquid from the column bottom part of the distillation column B (fraction B_{B}) was fed to the position 14.0 m away from the lowest part of the filling in the distillation column C as a liquid of 93°C. The distillation column was operated at a column top pressure of 0.0 kPaG, a column bottom pressure of 2.1 kPaG, a column bottom temperature of 127.5°C, and a reflux ratio of 2.0.

A liquid was continuously extracted with a pump at 0.38 kmol/h from the column bottom part of the distillation column C. In the liquid extracted from the column bottom part (fraction C_{B}), the flow rate was 0.01 kmol/h for EMC and 0.37 kmol/h for DEC, and the EMC content in the column bottom liquid was 1.5% by mass. In addition, a liquid (fraction C_{L}) was continuously extracted with a pump at 1.88 kmol/h from the column top part of the distillation column C. In the liquid extracted from the column top part (fraction C_{L}), the flow rate was 0.00012 kmol/h for DMC, 1.88 kmol/h for EMC, and 0.00005 kmol/h for DEC, so that the purification of EMC having a purity of 99.99% by mass or more was achieved.

### [Example 4]

The method for producing ethyl methyl carbonate was conducted using the apparatus shown in Figure 2. Using a distillation column made of SUS304 and having 19 m of a regular packing, MellaPak752Y as the distillation column A, the purification of EMC was carried out as follows. The separation capacity of the distillation column was 47 theoretical plates.

MeOH, EtOH, DMC, EMC, and DEC were respectively fed at 3.33 kmol/h, 1.74 kmol/h, 3.67 kmol/h, 2.08 kmol/h, and 0.26 kmol/h to the position 12.7 m away from the lowest part of the filling in the distillation column A as a liquid of 50°C. Similarly, DMC was fed at 0.408 kmol/h to the position 12.7 m away from the lowest part of the filling in the distillation column as a liquid of 50°C. In the feed liquid, the DMC concentration was 4.3 mol%, and the molar ratio of DMC to the total amount of EtOH and EMC was 0.11. The distillation column was continuously operated at a column top pressure of 40.4 kPaG, a column bottom pressure of 42.0 kPaG, a column bottom temperature of 119.2°C, and a reflux ratio of 1.2.

A liquid was continuously extracted with a pump at 2.21 kmol/h from the column bottom part of the distillation column A. In the liquid extracted from the column bottom part (fraction A_{B}), the flow rate was 0.00001 kmol/h for MtOH, 0.00004 kmol/h for EtOH, 0.00010 kmol/h for DMC, 1.95 kmol/h for EMC, and 0.26 kmol/h for DEC, and the total amount of ethanol and methanol to the EMC mass in the column bottom liquid was 11.7 ppm by mass, so that 20 ppm by mass or less was achieved. In addition, a liquid (fraction A_{L}) was continuously extracted with a pump at 8.87 kmol/h from the column top part of the distillation column A. In the liquid extracted from the column top part (fraction A_{L}), the flow rate was 3.33 kmol/h for MeOH, 1.74 kmol/h for EtOH, 3.670 kmol/h for DMC, and 0.130 kmol/h for EMC.

The extraction liquid from the column bottom part of the distillation column A (fraction A_{B}) was fed to the distillation column D and distilled as mentioned below.

The distillation column D was a distillation column made of SUS304 and having 47 m of a regular packing, MellaPak752Y, and the purification of EMC was carried out as follows. The separation capacity of the distillation column was 84 theoretical plates.

Gas was continuously extracted at 1.83 kmol/h from the position 26 m away from the lowest part of the filling in the distillation column D, condensed with a condenser, and then continuously extracted with a pump as the side cut (fraction D_{S}). In the liquid extracted from the side cut (fraction D_{S}), the flow rate was 0.00001 kmol/h for MtOH, 0.00003 kmol/h for EtOH, 0.0001 kmol/h for DMC, 1.82 kmol/h for EMC, and 0.00004 kmol/h for DEC, so that the purification of EMC having a purity of 99.99% by mass or more was achieved. In addition, a liquid (fraction D_{B}) was continuously extracted with a pump at 0.38 kmol/h from the column bottom part of the distillation column D. In the liquid extracted from the column bottom part (fraction D_{L}), the flow rate was 0.12 kmol/h for EMC and 0.26 kmol/h for DEC. Further, a liquid (fraction D_{T}) was continuously extracted with a pump at 0.0019 kmol/h from the column top part of the distillation column. In the liquid extracted from the column top part (fraction D_{T}), the flow rate was 0.000004 kmol/h for MtOH, 0.00001 kmol/h for EtOH, and 0.002 kmol/h for EMC.

### [Example 5]

The method for producing ethyl methyl carbonate was conducted using the apparatus shown in Figure 3. The operation conditions and flow rate of the distillation column A, distillation column B, and distillation column C were the same as those in Example 1. The extraction liquid (fraction C_{B}) from the column bottom part of the distillation column C was fed to the distillation column F and distilled as mentioned below.

The distillation column F was a distillation column made of SUS304 and having 46 m of a regular packing, MellaPak250Y, and the purification of DEC was carried out as follows. The separation capacity of the distillation column was 50 theoretical plates.

Gas was continuously extracted at 0.37 kmol/h from the position 22 m away from the lowest part of the filling in the distillation column F, condensed with a condenser, and then continuously extracted with a pump as the side cut (fraction F_{S}). In the liquid extracted from the side cut (fraction F_{S}), the flow rate was 0.00002 kmol/h for EMC and 0.37 kmol/h for DEC, so that the purification of DEC having a purity of 99.99% by mass or more was achieved. In addition, a liquid (fraction F_{B}) was continuously extracted with a pump at 0.0001 kmol/h from the column bottom part of the distillation column F. In the liquid extracted from the column bottom part (fraction F_{B}), the flow rate of DEC was 0.0001 kmol/h. Further, a liquid (fraction F_{L}) was continuously extracted with a pump at 0.06 kmol/h from the column top part of the distillation column F. In the liquid extracted from the column top part (fraction F_{L}), the flow rate was 0.06 kmol/h for EMC and 0.00002 kmol/h for DEC.

### [Example 6]

The method for producing ethyl methyl carbonate was conducted using the apparatus shown in Figure 4. The operation conditions and flow rate of the distillation column A and distillation column D were the same as those in Example 2.

The extraction liquid (fraction D_{B}) from the column bottom part of the distillation column D was fed to the distillation column E and distilled as mentioned below.

The distillation column E was a distillation column made of SUS304 and having 46 m of a regular packing, MellaPak250Y, and the purification of DEC was carried out as follows. The separation capacity of the distillation column was 50 theoretical plates.

A liquid (fraction E_{B}) was continuously extracted with a pump at 0.26 kmol/h from the column bottom part of the distillation column E. In the liquid extracted from the column bottom part (fraction E_{B}), the flow rate was 0.000003 kmol/h for EMC and 0.26 kmol/h for DEC. Further, a liquid (fraction E_{L}) was continuously extracted with a pump at 0.13 kmol/h from the column top part of the distillation column. In the liquid extracted from the column top part (fraction E_{L}), the flow rate was 0.13 kmol/h for EMC and 0.000003 kmol/h for DEC.

### [Example 7]

The method for producing ethyl methyl carbonate was conducted using the apparatus shown in Figure 5. DMC, EtOH, and a 3.2% by mass (1.9 mol%) solution of sodium methoxide in methanol were respectively fed to a stirred tank reactor having a capacity of 1.5 m³ at a flow rate of 475.2 kg/h (5.28 kmol/h), 210.7 kg/h (4.58 kmol/h), and 4.3 kg/h (0.13 kmol/h), and reacted under the conditions of 50°C and 103 kPaG, and the reactant was extracted from the stirred tank reactor at 690 kg/h (9.99 kmol/h) to obtain a reaction composition containing EMC.

The reaction composition was filtered, then fed to a stirred tank holding 20 kg of a solid adsorbent, stirred for 1 hour, then filtered, and fed to the distillation column A, followed by distillation as mentioned below.

In a distillation column made of carbon steel and having 60 porous plate trays, the purification of EMC was carried out as follows. The separation capacity of the distillation column was 39 theoretical plates.

MeOH, EtOH, DMC, EMC, and DEC were respectively fed at 2.85 kmol/h, 1.86 kmol/h, 2.93 kmol/h, 1.98 kmol/h, and 0.37 kmol/h onto the 46th tray from the bottom of the distillation column as a liquid of 50°C. In the feed liquid, the DMC concentration was 29.3 mol%, and the molar ratio of DMC to the total amount of EtOH and EMC was 0.76. The distillation column A was continuously operated at a column top pressure of 0.0 kPaG, a column bottom temperature of 3.1 kPaG, a column bottom temperature of 109.2°C, and a reflux ratio of 1.7.

A liquid was continuously extracted with a pump at 2.36 kmol/h from the column bottom part of the distillation column A. In the liquid extracted from the column bottom part (fraction A_{B}), the flow rate was 0.00 mol/h for EtOH, 0.01 kmol/h for DMC, 1.98 kmol/h for EMC, and 0.37 kmol/h for DEC, and the total amount of ethanol and methanol to the EMC mass in the column bottom liquid was 0 ppm by mass, so that 20 ppm by mass or less was achieved. In addition, a liquid was continuously extracted with a pump at 7.63 kmol/h from the column top part of the distillation column A. In the liquid extracted from the column top part (fraction A_{L}), the flow rate was 2.85 kmol/h for MeOH, 1.86 kmol/h for EtOH, 2.92 kmol/h for DMC, and 0.001 kmol/h for EMC.

The extraction liquid from the column bottom part of the distillation column A (fraction A_{B}) was fed to the distillation column B and distilled as mentioned below.

The distillation column B was a distillation column made of SUS304 and having 27 m of a regular packing, MellaPak752Y, and the purification of EMC was carried out as follows. The separation capacity of the distillation column was 48 theoretical plates.

The extraction liquid from the column bottom part of the distillation column A (fraction A_{B}) was fed to the position 13.0 m away from the lowest part of the filling in the distillation column B as a liquid of 107°C. The distillation column B was operated at a column top pressure of -40.0 kPaG, a column bottom pressure of -37.9 kPaG, a column bottom temperature of 93.7°C, and a reflux ratio of 1,100.

A liquid was continuously extracted with a pump at 2.35 kmol/h from the column bottom part of the distillation column B. In the liquid extracted from the column bottom part (fraction B_{B}), the flow rate was 0.00007 kmol/h for DMC, 1.98 kmol/h for EMC, and 0.37 kmol/h for DEC, and the DMC content in the column bottom liquid was 25 ppm by mass, so that 50 ppm by mass or less was achieved. In addition, a liquid was continuously extracted with a pump at 0.01 kmol/h from the column top part of the distillation column B. In the liquid extracted from the column top part (fraction B_{L}), the flow rate was 0.01 kmol/h for DMC and 0.0016 kmol/h for EMC.

The extraction liquid from the column bottom part of the distillation column B (fraction B_{B}) was fed to the distillation column D and distilled as mentioned below.

The distillation column D was a distillation column made of SUS304 and having 47 m of a regular packing, MellaPak752Y, and the purification of EMC was carried out as follows. The separation capacity of the distillation column was 72 theoretical plates.

Gas was continuously extracted at 1.92 kmol/h from the position 26 m away from the lowest part of the filling in the distillation column D, condensed with a condenser, and then continuously extracted with a pump as the side cut. In the liquid extracted from the side cut (fraction D_{S}), the flow rate was 0.00002 kmol/h for DMC, 1.92 kmol/h for EMC, and 0.00008 kmol/h for DEC, so that the purification of EMC having a purity of 99.99% by mass or more was achieved. In addition, a liquid (fraction D_{B}) was continuously extracted with a pump at 0.43 kmol/h from the column bottom part of the distillation column D. In the liquid extracted from the column bottom part (fraction D_{B}), the flow rate was 0.06 kmol/h for EMC and 0.37 kmol/h for DEC. Further, a liquid (fraction D_{L}) was continuously extracted with a pump at 0.0011 kmol/h from the column top part of the distillation column D. In the liquid extracted from the column top part (fraction D_{L}), the flow rate was 0.00005 kmol/h for DMC and 0.0010 kmol/h for EMC.

### [Comparative Example 1]

In a distillation column made of carbon steel and having 60 porous plate trays, the purification of EMC was carried out as follows. The separation capacity of the distillation column was 39 theoretical plates.

MeOH, EtOH, EMC, and DEC were respectively fed at 2.85 kmol/h, 1.86 kmol/h, 1.98 kmol/h, and 0.37 kmol/h onto the 46th tray from the bottom of the distillation column as a liquid of 50°C. They were fed onto the 46th tray from the bottom of the distillation column as a liquid of 50°C. In the feed liquid, the DMC concentration was 0 mol%, and the molar ratio of DMC to the total amount of EtOH and EMC was 0. The distillation column was continuously operated at a column top pressure of 0.0 kPaG, a column bottom pressure of 7.1 kPaG, a column bottom temperature of 100.2°C, and a reflux ratio of 10.0.

A liquid was continuously extracted with a pump at 2.59 kmol/h from the column bottom part of the distillation column. In the liquid extracted from the column bottom part, the flow rate was 0.00000 mol/h for MeOH, 0.25329 kmol/h for EtOH, 1.96 kmol/h for EMC, and 0.37 kmol/h for DEC, and the total amount of ethanol and methanol to the EMC mass in the column bottom liquid was 57,042 ppm by mass, so that 20 ppm by mass or less was not achieved. In addition, a liquid was continuously extracted with a pump at 4.47 kmol/h from the column top part of the distillation column. In the liquid extracted from the column top part, the flow rate was 2.85 kmol/h for MeOH, 1.61 kmol/h for EtOH, and 0.016 km for EMC.

### [Comparative Example 2]

In a distillation column made of SUS304 and having 19 m of a regular packing, MellaPak752Y, the purification of EMC was carried out as follows. The separation capacity of the distillation column was 47 theoretical plates.

MeOH, EtOH, EMC, and DEC were respectively fed at 3.33 kmol/h, 1.74 kmol/h, 2.08 kmol/h, and 0.26 kmol/h to the position 12.7 m away from the lowest part of the filling in the distillation column as a liquid of 50°C. In the feed liquid, the DMC concentration was 0.00 kmol%, and the molar ratio of DMC to the total amount of EtOH and EMC was 0.00. The distillation column was continuously operated at a column top pressure of 2.5 kPaG, a column bottom pressure of 5.1 kPaG, a column bottom temperature of 91.1°C, and a reflux ratio of 1.0.

A liquid was continuously extracted with a pump at 2.55 kmol/h from the column bottom part of the distillation column. In the liquid extracted from the column bottom part, the flow rate was 0.00000 kmol/h for MeOH, 0.40097 kmol/h for EtOH, 1.89 kmol/h for EMC, and 0.26 kmol/h for DEC, and the total amount of ethanol and methanol to the EMC mass in the column bottom liquid was 93,758 ppm by mass, and 20 ppm by mass or less was not achieved. In addition, a liquid was continuously extracted with a pump at 4.85 kmol/h from the column top part of the distillation column. In the liquid extracted from the column top part, the flow rate was 3.33 kmol/h for MeOH, 1.34 kmol/h for EtOH, and 0.185 kmol/h for EMC.

### [Comparative Example 3]

In a distillation column made of SUS304 and having 20 porous trays, the purification of EMC was carried out as follows. The separation capacity of the distillation column was 13 theoretical plates.

MeOH, EtOH, DMC, EMC, and DEC were respectively fed at 2.85 kmol/h, 1.86 kmol/h, 2.93 kmol/h, 1.98 kmol/h, and 0.37 kmol/h onto the 46th tray from the bottom of the distillation column as a liquid of 50°C. In the feed liquid, the DMC concentration was 29.3 mol%, and the molar ratio of DMC to the total amount of EtOH and EMC was 0.76. The distillation column was continuously operated at a column top pressure of 0.0 kPaG, a column bottom pressure of 1.0 kPaG, a column bottom temperature of 105.7°C, and a reflux ratio of 1.7.

A liquid was continuously extracted with a pump at 5.48 kmol/h from the column bottom part of the distillation column. In the liquid extracted from the column bottom part, the flow rate was 0.03 kmol/h for MeOH, 0.990194 kmol/h for EtOH, 2.13 kmol/h for EMC, and 0.37 kmol/h for DEC, and the total amount of ethanol and methanol to the EMC mass in the column bottom liquid was 228,767 ppm by mass, so that 20 ppm by mass or less was not achieved. In addition, a liquid was continuously extracted with a pump at 4.51 kmol/h from the column top part of the distillation column. In the liquid extracted from the column top part, the flow rate was 2.82 kmol/h for MeOH, 0.87 kmol/h for EtOH, 0.80 for DMC, and 0.023 kmol/h for EMC.

The above results are summarized and shown in Tables 1 to 3.

**[Table 1-1]**

| Table 1 (1/4) | | | | | | |
|---|---|---|---|---|---|---|
| | | | Example 1 | Example 3 | Example 5 | Comparative Example 3 |
| External addition | Amount of DMC added | kmol/h | - | - | - | - |
| | Flow rate | kmol/h | 9.99 | 9.99 | 9.99 | 9.99 |
| | MeOH flow rate | kmol/h | 2.85 | 2.85 | 2.85 | 2.85 |
| | EtOH flow rate | kmol/h | 1.86 | 1.86 | 1.86 | 1.86 |
| Distillation column A | DMC flow rate | kmol/h | 2.93 | 2.93 | 2.93 | 2.93 |
| | EMC flow rate | kmol/h | 1.98 | 1.98 | 1.98 | 1.98 |
| Feed | DEC flow rate | kmol/h | 0.37 | 0.37 | 0.37 | 0.37 |
| | DMC concentration | mol% | 29.3 | 29.3 | 29.3 | 29.3 |
| | EMC concentration | mol% | 19.8 | 19.8 | 19.8 | 19.8 |
| | DMC/(EtOH+EMC) | | 0.76 | 0.76 | 0.76 | 0.76 |
| | Temperature | °C | 50 | 50 | 50 | 50 |
| | Flow rate | kmol/h | 7.63 | 7.39 | 7.63 | 4.51 |
| Distillation column A | MeOH flow rate | kmol/h | 2.85 | 2.85 | 2.85 | 2.82 |
| | EtOH flow rate | kmol/h | 1.86 | 1.86 | 1.86 | 0.87 |
| Column top | DMC flow rate | kmol/h | 2.92 | 2.59 | 2.92 | 0.80 |
| Fraction A_{L} | EMC flow rate | kmol/h | 0.001 | 0.093 | 0.001 | 0.023 |
| | DEC flow rate | kmol/h | 0.00 | 0.00 | 0.00 | 0.00 |

**[Table 1-2]**

| Table 1 (2/4) | | | | | | |
|---|---|---|---|---|---|---|
| | | | Example 1 | Example 3 | Example 5 | Comparative Example 3 |
| | Flow rate | kmol/h | 2.36 | 2.60 | 2.36 | 5.48 |
| Distillation column A | MeOH flow rate | kmol/h | 0.00 | 0.00 | 0.00 | 0.03 |
| | EtOH flow rate | kmol/h | 0.000000 | 0.000003 | 0.000000 | 0.990194 |
| | DMC flow rate | kmol/h | 0.01 | 0.34 | 0.01 | 2.13 |
| Columnbottom | EMC flow rate | kmol/h | 1.98 | 1.89 | 1.98 | 1.96 |
| Fraction A_{B} | DEC flow rate | kmol/h | 0.37 | 0.37 | 0.37 | 0.37 |
| | (MeOH+EtOH)/EMC | ppm by mass | 0 | 1 | 0 | 228,767 |
| | Internal | - | Porous plate tray 60 plates | Porous plate tray 20 plates | Porous plate tray 60 plates | Porous plate tray 20 plates |
| | Separation capacity | - | 39 theoretical plates | 13 theoretical plates | 39 theoretical plates | 13 theoretical plates |
| Distillation column A | Column bottom temperature | °C | 109.2 | 105.7 | 109.2 | 105.7 |
| | Column top pressure | kPaG | 0.0 | 0.0 | 0.0 | 0.0 |
| Conditions | Column bottom pressure | kPaG | 3.1 | 1.0 | 3.1 | 1.0 |
| | Loss of EMC (Column top EMC) | kmol/h | 0.001 | 0.093 | 0.020 | 0.023 |
| | EMC yield | % | 99.9 | 95.3 | 99.0 | 98.9 |
| | Amount of heat | kW | 229 | 245 | 229 | 142 |
| | Reflux ratio | - | 1.7 | 2.0 | 1.7 | 1.7 |

**[Table 1-3]**

| Table 1 (3/4) | | | | | | |
|---|---|---|---|---|---|---|
| | | | Example 1 | Example 3 | Example 5 | Comparative Example 3 |
| Distillation column B | Flow rate | kmol/h | 0.01 | 0.34 | 0.0116 | 2.10 |
| | MeOH flow rate | kmol/h | 0.00 | 0.00 | 0.00 | 0.03 |
| | EtOH flow rate | kmol/h | 0.00 | 0.00 | 0.00 | 0.99 |
| Column top | DMC flow rate | kmol/h | 0.01 | 0.34 | 0.01 | 2.10 |
| Fraction B_{L} | EMC flow rate | kmol/h | 0.002 | 0.001 | 0.002 | 0.000 |
| | DEC flow rate | kmol/h | 0.00 | 0.00 | 0.00 | 0.00 |
| Distillation column B | Flow rate | kmol/h | 2.35 | 2.26 | 2.35 | 2.35 |
| | DMC flow rate | kmol/h | 0.0001 | 0.0001 | 0.0001 | 0.0254 |
| Column bottom | EMC flow rate | kmol/h | 1.98 | 1.89 | 1.98 | 1.96 |
| Fraction B_{B} | DEC flow rate | kmol/h | 0.37 | 0.37 | 0.37 | 0.37 |
| | Internal | - | MellaPak752Y | MellaPak752Y | MellaPak752Y | MellaPak752Y |
| | | | 27m | 27m | 27m | 27m |
| Distillation column B | Separation capacity | - | 48 theoretical plates | 48 theoretical plates | 48 theoretical plates | 48 theoretical plates |
| | Column bottom temperature | °C | 93.7°C | 93.7°C | 93.7°C | 93.7°C |
| Conditions | Column top pressure | kPaG | -40.0 | -40.0 | -40.0 | -40.0 |
| | Column bottom pressure | kPaG | -37.9 | -37.9 | -37.9 | -37.9 |
| | Amount of heat | kW | 25 | 76 | 25 | 353 |
| | Reflux ratio | - | 1,100 | 20 | 1,100 | 10 |

**[Table 1-4]**

| Table 1 (4/4) | | | | | | |
|---|---|---|---|---|---|---|
| | | | Example 1 | Example 3 | Example 5 | Comparative Example 3 |
| Distillation column C | Flow rate | kmol/h | 1.92 | 1.88 | 1.92 | 1.92 |
| | DMC flow rate | kmol/h | 0.00007 | 0.00012 | 0.00007 | 0.02540 |
| Column top | EMC flow rate | kmol/h | 1.92 | 1.88 | 1.92 | 1.90 |
| Fraction C_{L} | DEC flow rate | kmol/h | 0.00008 | 0.00005 | 0.00008 | 0.00003 |
| Distillation column C | Flow rate | kmol/h | 0.43 | 0.38 | 0.43 | 0.43 |
| | DMC flow rate | kmol/h | 0.00 | 0.00 | 0.00 | 0.00 |
| Column bottom | EMC flow rate | kmol/h | 0.06 | 0.01 | 0.06 | 0.06 |
| Fraction C_{B} | DEC flow rate | kmol/h | 0.37 | 0.37 | 0.37 | 0.37 |
| Distillation column C Conditions | Internal | - | MellaPak250Y | MellaPak250Y | MellaPak250Y | MellaPak250Y |
| | | | 49m | 49m | 49m | 49m |
| | Separation capacity | - | 47 theoretical plates | 47 theoretical plates | 47 theoretical plates | 47 theoretical plates |
| | Column bottom temperature | °C | 127.5 | 127.5 | 127.5 | 127.5 |
| | Column top pressure | kPaG | 0.0 | 0.0 | 0.0 | 0.0 |
| | Column bottom pressure | kPaG | 2.1 | 2.1 | 2.1 | 2.1 |
| | Amount of heat | kW | 64 | 62 | 64 | 69 |
| | Reflux ratio | - | 2.0 | 2.0 | 2.0 | 2.3 |
| Distillation column F | | | No | No | Yes | No |
| Distillation step EMC yield | | % | 97.0 | 94.9 | 97.0 | 95.9 |
| EMC purity after purification | | % by mass | 99.9922 | 99.9911 | 99.9922 | 98.8541 |
| Amount of heat in distillation step/Article EMC | | kW/kg | 1.59 | 1.96 | 1.59 | 2.86 |

**[Table 2-1]**

| Table 2 (1/4) | | | | | | | |
|---|---|---|---|---|---|---|---|
| | | | Example 2 | Example 4 | Example 6 | Comparative Example 1 | Comparative Example 2 |
| External addition | Amount of DMC added | kmol/h | 0.408 | 0.408 | 0.408 | - | - |
| Distillation column A Feed | Flow rate | kmol/h | 11.07 | 11.07 | 11.07 | 7.06 | 7.40 |
| | MeOH flow rate | kmol/h | 3.33 | 3.33 | 3.33 | 2.85 | 3.33 |
| | EtOH flow rate | kmol/h | 1.74 | 1.74 | 1.74 | 1.86 | 1.74 |
| | DMC flow rate | kmol/h | 3.67 | 3.67 | 3.67 | 0.00 | 0.00 |
| | EMC flow rate | kmol/h | 2.08 | 2.08 | 2.08 | 1.98 | 2.08 |
| | DEC flow rate | kmol/h | 0.26 | 0.26 | 0.26 | 0.37 | 0.26 |
| | DMC concentration | mol% | 4.3 | 4.3 | 4.3 | 0.0 | 0.0 |
| | EMC concentration | mol% | 26.6 | 26.6 | 26.6 | 28.0 | 28.1 |
| | DMC/(EtOH+EMC) | | 0.11 | 0.11 | 0.11 | 0.00 | 0.00 |
| | Temperature | °C | 50 | 50 | 50 | 50 | 50 |
| Distillation column A | Flow rate | kmol/h | 8.69 | 8.87 | 8.69 | 4.47 | 4.85 |
| | MeOH flow rate | kmol/h | 3.33 | 3.33 | 3.33 | 2.85 | 3.33 |
| | EtOH flow rate | kmol/h | 1.74 | 1.74 | 1.74 | 1.61 | 1.34 |
| Column top | DMC flow rate | kmol/h | 3.61 | 3.670 | 3.61 | 0.00 | 0.00 |
| Fraction A_{L} | EMC flow rate | kmol/h | 0.018 | 0.130 | 0.018 | 0.016 | 0.185 |
| | DEC flow rate | kmol/h | 0.00 | 0.00 | 0.00 | 0.00 | 0.00 |

**[Table 2-2]**

| Table 2 (2/4) | | | | | | | |
|---|---|---|---|---|---|---|---|
| | | | Example 2 | Example 4 | Example 6 | Comparative Example 1 | Comparative Example 2 |
| | Flow rate | kmol/h | 2.38 | 2.21 | 2.38 | 2.59 | 2.55 |
| Distillation column A | MeOH flow rate | kmol/h | 0.00000 | 0.00001 | 0.00000 | 0.00000 | 0.00000 |
| | EtOH flow rate | kmol/h | 0.00000 | 0.00004 | 0.00000 | 0.25329 | 0.40097 |
| Column bottom | DMC flow rate | kmol/h | 0.06000 | 0.00010 | 0.06000 | 0.00000 | 0.00000 |
| | EMC flow rate | kmol/h | 2.06 | 1.95 | 2.06 | 1.96 | 1.89 |
| Fraction | DEC flow rate | kmol/h | 0.26 | 0.26 | 0.26 | 0.37 | 0.26 |
| A_{B} | (MeOH+EtOH)/EMC | ppm by mass | 0 | 12 | 0 | 57,042 | 93,758 |
| | Internal | - | MellaPak752Y | MellaPak752Y | MellaPak752Y | Porous plate tray | MellaPak752 Y |
| | | | 19m | 19m | 19m | 60 plates | 19m |
| | Separation capacity | - | 47 theoretical plates | 47 theoretical plates | 47 theoretical plates | 39 theoretical plates | 47 theoretical plates |
| Distillation column A | Column bottom temperature | °C | 107.8 | 119.2 | 107.8 | 100.2 | 91.6 |
| | Column top pressure | kPaG | 2.3 | 40.4 | 2.3 | 0 | 2.5 |
| Conditions | Column bottom pressure | kPaG | 3.8 | 42.0 | 3.8 | 7.1 | 5.1 |
| | Loss of EMC (Column top EMC) | kmol/h | 0.018 | 0.130 | 0.121 | 0.016 | 0.185 |
| | EMC yield | % | 99.1 | 93.9 | 94.3 | 99.2 | 91.1 |
| | Amount of heat | kW | 200 | 215 | 200 | 522 | 114 |
| | Reflux ratio | - | 1.0 | 1.2 | 1.0 | 10.0 | 1.0 |

**[Table 2-3]**

| Table 2 (3/4) | | | | | | | |
|---|---|---|---|---|---|---|---|
| | | | Example 2 | Example 4 | Example 6 | Comparative Example 1 | Comparative Example 2 |
| | Flow rate | kmol/h | 0.062 | 0.0019 | 0.062 | 0.0007 | 0.0007 |
| Distillation column D | MeOH flow rate | kmol/h | 0.000000 | 0.000004 | 0.000000 | 0.000000 | 0.000000 |
| | EtOH flow rate | kmol/h | 0.00000 | 0.00001 | 0.00000 | 0.00266 | 0.00279 |
| Column top | DMC flow rate | kmol/h | 0.06 | 0.00 | 0.06 | 0.00 | 0.00 |
| Fraction D_{L} | EMC flow rate | kmol/h | 0.002 | 0.002 | 0.002 | 0.001 | 0.001 |
| | DEC flow rate | kmol/h | 0.00 | 0.00 | 0.00 | 0.00 | 0.00 |
| | Flow rate | kmol/h | 1.92 | 1.83 | 1.92 | 1.72 | 1.65 |
| Distillation column D | MeOH flow rate | kmol/h | 0.00000 | 0.00001 | 0.00000 | 0.00000 | 0.00000 |
| | EtOH flow rate | kmol/h | 0.00000 | 0.00003 | 0.00000 | 0.24804 | 0.39721 |
| Side cut | DMC flow rate | kmol/h | 0.0001 | 0.0001 | 0.0001 | 0.0000 | 0.0000 |
| Fraction D_{S} | EMC flow rate | kmol/h | 1.92 | 1.82 | 1.92 | 1.72 | 1.65 |
| | DEC flow rate | kmol/h | 0.00005 | 0.00004 | 0.00005 | 0.00001 | 0.00000 |

**[Table 2-4]**

| Table 2 (4/4) | | | | | | | |
|---|---|---|---|---|---|---|---|
| | | | Example 2 | Example 4 | Example 6 | Comparative Example 1 | Comparative Example 2 |
| Distillation column D | Flow rate | kmol/h | 0.39 | 0.38 | 0.39 | 0.62 | 0.50 |
| | EtOH flow rate | kmol/h | 0.00 | 0.00 | 0.00 | 0.00 | 0.00 |
| Column bottom | DMC flow rate | kmol/h | 0.00 | 0.00 | 0.00 | 0.00 | 0.00 |
| | EMC flow rate | kmol/h | 0.13 | 0.12 | 0.13 | 0.25 | 0.24 |
| Fraction | DEC flow rate | kmol/h | 0.26 | 0.26 | 0.26 | 0.37 | 0.26 |
| D_{B} | | | | | | | |
| | Internal | - | MellaPak752Y | MellaPak752Y | MellaPak752Y | MellaPak752Y | MellaPak752Y |
| | | | 47m | 47m | 47m | 47m | |
| | Separation capacity | - | 84 theoretical plates | 84 theoretical plates | 84 theoretical plates | 84 theoretical plates | 84 theoretical plates |
| Distillation column D | Column bottom temperature | °C | 127.8 | 127.8 | 127.8 | 127.8 | 127.8 |
| Conditions | Column top pressure | kPaG | 0 | 0 | 0 | 0 | 0 |
| | Column bottom pressure | kPaG | 2.9 | 2.9 | 2.9 | 2.9 | 2.9 |
| | Amount of heat | kW | 51 | 51 | 51 | 56 | 56 |
| | Reflux ratio | - | 1,300 | 1,300 | 1,300 | 720 | 720 |
| Distillation column E | | | No | No | Yes | No | No |
| Distillation step EMC yield | | % | 92.6 | 87.9 | 92.6 | 86.6 | 79.4 |
| EMC purity after purification | | % by mass | 99.9929 | 99.9922 | 99.9929 | 93.9846 | 90.3680 |
| Amount of heat in distillation step/Article EMC | | kW/kg | 1.25 | 1.40 | 1.25 | 3.24 | 0.99 |

**[Table 3-1]**

| Table 3 (1/3) | | | |
|---|---|---|---|
| | | | Example 7 |
| External addition | Amount of DMC added | kmol/h | - |
| | Flow rate | kmol/h | 9.99 |
| | MeOH flow rate | kmol/h | 2.85 |
| | EtOH flow rate | kmol/h | 1.86 |
| | DMC flow rate | kmol/h | 2.93 |
| Distillation column A | EMC flow rate | kmol/h | 1.98 |
| Feed | DEC flow rate | kmol/h | 0.37 |
| | DMC concentration | mol% | 29.3 |
| | EMC concentration | mol% | 19.8 |
| | DMC/(EtOH+EMC) | | 0.76 |
| | Temperature | °C | 50 |
| | Flow rate | kmol/h | 7.63 |
| Distillation column A | MeOH flow rate | kmol/h | 2.85 |
| | EtOH flow rate | kmol/h | 1.86 |
| Column top | DMC flow rate | kmol/h | 2.92 |
| Fraction A_{L} | EMC flow rate | kmol/h | 0.001 |
| | DEC flow rate | kmol/h | 0.00 |
| | Flow rate | kmol/h | 2.36 |
| | MeOH flow rate | kmol/h | 0.00 |
| Distillation column A | EtOH flow rate | kmol/h | 0.00 |
| Column bottom | DMC flow rate | kmol/h | 0.01 |
| Fraction A_{B} | EMC flow rate | kmol/h | 1.98 |
| | DEC flow rate | kmol/h | 0.37 |
| | (MeOH+EtOH)/EMC | ppm by mass | 0 |
| | Internal | - | Porous plate tray |
| | | | 60 plates |
| | Separation capacity | - | 39 theoretical plates |
| | Column bottom temperature | °C | 109.2 |
| Distillation column A | Column top pressure | kPaG | 0.0 |
| Conditions | Column bottom pressure | kPaG | 3.1 |
| | Loss of EMC (Column top EMC) | kmol/h | 0.001 |
| | EMC yield | % | 99.9 |
| | Amount of heat | kW | 229 |
| | Reflux ratio | - | 1.7 |

**[Table 3-2]**

| Table 3 (2/3) | | | |
|---|---|---|---|
| | | | Example 7 |
| | Flow rate | kmol/h | 0.01 |
| Distillation column B | MeOH flow rate | kmol/h | 0.00 |
| | EtOH flow rate | kmol/h | 0.00 |
| Column top | DMC flow rate | kmol/h | 0.01 |
| Fraction B_{L} | EMC flow rate | kmol/h | 0.0016 |
| | DEC flow rate | kmol/h | 0.00 |
| Distillation column B | Flow rate | kmol/h | 2.35 |
| | DMC flow rate | kmol/h | 0.00007 |
| Column bottom | EMC flow rate | kmol/h | 1.98 |
| Fraction B_{B} | DEC flow rate | kmol/h | 0.37 |
| | Internal | - | MellaPak752Y |
| | | | 27m |
| | Separation capacity | - | 48 theoretical plates |
| Distillation column B | Column bottom temperature | °C | 93.7 |
| Conditions | Column top pressure | kPaG | -40.0 |
| | Column bottom pressure | kPaG | -37.9 |
| | Amount of heat | kW | 25 |
| | Reflux ratio | - | 1,100 |
| | Flow rate | kmol/h | 0.0011 |
| Distillation column D | MeOH flow rate | kmol/h | 0.00001 |
| | EtOH flow rate | kmol/h | 0.00000 |
| Column top | DMC flow rate | kmol/h | 0.00005 |
| Fraction D_{L} | EMC flow rate | kmol/h | 0.0010 |
| | DEC flow rate | kmol/h | 0.0000 |
| | Flow rate | kmol/h | 1.92 |
| | MeOH flow rate | kmol/h | 0.00 |
| Distillation column D | EtOH flow rate | kmol/h | 0.00 |
| Side cut | DMC flow rate | kmol/h | 0.00002 |
| Fraction D_{S} | EMC flow rate | kmol/h | 1.92 |
| | DEC flow rate | kmol/h | 0.00008 |

**[Table 3-3]**

| Table 3 (3/3) | | | |
|---|---|---|---|
| | | | Example 7 |
| Distillation column D Column bottom Fraction D_{B} | Flow rate | kmol/h | 0.43 |
| | EtOH flow rate | kmol/h | 0.00 |
| | DMC flow rate | kmol/h | 0.00 |
| | EMC flow rate | kmol/h | 0.06 |
| | DEC flow rate | kmol/h | 0.37 |
| Distillation column D Conditions | Internal | - | MellaPak752Y |
| | | | 47m |
| | Separation capacity | - | 72 theoretical plates |
| | Column bottom temperature | °C | 127.8 |
| | Column top pressure | kPaG | 0.0 |
| | Column bottom pressure | kPaG | 2.9 |
| | Amount of heat | kW | 45 |
| | Reflux ratio | - | 3817 |
| Distillation step EMC yield | | % | 97.0 |
| EMC purity after purification | | % by mass | 99.9943 |
| Amount of heat in distillation step/Article EMC | | kW/kg | 1.50 |

It has been found from the examination of Examples and Comparative Examples described above that the method for producing ethyl methyl carbonate according to the present embodiment enables the amount of heat required for the production of ethyl methyl carbonate in the distillation step to be kept low and enables ethyl methyl carbonate having low ethanol and methanol contents to be produced with high efficiency.

### Reference Signs List

A, B, C, D, E, F distillation column
r reboiler
R reaction apparatus
11 reaction vessel
12 raw material feed part
13 stirring apparatus
14 extraction part

## Claims

1. A method for producing ethyl methyl carbonate comprising:
distillation step A of feeding a feed containing dimethyl carbonate, ethyl methyl carbonate, and ethanol to a distillation column A, and extracting a fraction A_{B} from a column bottom of the distillation column A, wherein
the feed contains dimethyl carbonate in an amount of 25 mol% or more based on a whole amount of the feed and ethyl methyl carbonate in an amount of 12 mol% or more based on a whole amount of the feed,
the feed has a molar ratio dimethyl carbonate/(ethanol + ethyl methyl carbonate) of 0.1 to 2.0, and
a proportion of ethanol to ethyl methyl carbonate in the fraction A_{B} is 20 ppm by mass or less, and a proportion of methanol to ethyl methyl carbonate in the fraction A_{B} is 20 ppm by mass or less.

2. The method for producing ethyl methyl carbonate according to claim 1, further comprising:
(1) distillation step B of feeding the fraction A_{B} to a distillation column B, and extracting a fraction B_{B} from which a low-boiling point substance having a lower boiling point than a boiling point of ethyl methyl carbonate has been removed, from a column bottom of the distillation column B, and
distillation step C of feeding the fraction B_{B} to a distillation column C, and extracting a fraction C_{L} from a column upper part of the distillation column C,
wherein ethyl methyl carbonate purity in the fraction C_{L} is 99.99% by mass or more,
or
(2) distillation step D of feeding the fraction A_{B} to a distillation column D, and extracting a fraction D_{B} from a column bottom of the distillation column D, a fraction D_{L} from a column top, and a fraction D_{S} from an intermediate part of the distillation column D,
wherein ethyl methyl carbonate purity in the fraction D_{S} is 99.99% by mass or more,
or
(3) distillation step B of feeding the fraction A_{B} to a distillation column B, and extracting a fraction B_{B} from which a low-boiling point substance having a lower boiling point than a boiling point of ethyl methyl carbonate has been removed, from a column bottom of the distillation column B, and
distillation step D of feeding the fraction B_{B} to a distillation column D, and extracting a fraction D_{B} from a column bottom of the distillation column D, a fraction D_{L} from a column top, and a fraction D_{S} from an intermediate part of the distillation column D,
wherein ethyl methyl carbonate purity in the fraction D_{S} is 99.99% by mass or more.

3. The method for producing ethyl methyl carbonate according to claim 1, wherein a column top pressure in the distillation column A is 0 kPaG to 50 kPaG.

4. The method for producing ethyl methyl carbonate according to claim 1, wherein the method for feeding at least a part of dimethyl carbonate in the feed to the distillation column A is a single addition of a liquid with a pump.

5. The method for producing ethyl methyl carbonate according to claim 1, wherein the feed is introduced from a column intermediate part of the distillation column A.

6. The method for producing ethyl methyl carbonate according to claim 1, wherein the feed contains ethanol in an amount of 0.5 mol% or more based on a whole amount of the feed.

7. The method for producing ethyl methyl carbonate according to claim 1, wherein the distillation column A includes a tray and/or a filling as an internal.

8. The method for producing ethyl methyl carbonate according to any one of claims 1 to 7, further comprising, before the distillation step A:
a reaction step of subjecting dimethyl carbonate and ethanol to a transesterification reaction in a reaction apparatus to obtain a reaction composition,
wherein the reaction composition is to be fed to the distillation column A as at least a part of the feed.

9. The method for producing ethyl methyl carbonate according to claim 2, further comprising a step of distilling a fraction C_{B} or a fraction D_{B} extracted from a column bottom of the distillation column C or the distillation column D to obtain diethyl carbonate having a purity of 99.99% by mass or more.

10. The method for producing ethyl methyl carbonate according to any one of claims 1 to 7, further comprising:
before the distillation step A, a reaction step of subjecting dimethyl carbonate and ethanol to a transesterification reaction in a reaction apparatus to obtain a reaction composition,
distillation step B of feeding the fraction A_{B} to a distillation column B, and extracting a fraction B_{B} from which a low-boiling point substance having a lower boiling point than a boiling point of ethyl methyl carbonate has been removed, from a column bottom of the distillation column B, and
distillation step C of feeding the fraction B_{B} to a distillation column C, and extracting a fraction C_{L} from a column upper part of the distillation column C,
wherein the reaction composition is fed to the distillation column A as at least a part of the feed, and
ethyl methyl carbonate purity in the fraction C_{L} is 99.99% by mass or more.
